# EUROPEAN PATENT SPECIFICATION

(11) **EP 2 567 667 B1**
(45) Date of publication and mention of the grant of the patent: **16.04.2014**
(21) Application number: 11837840.5
(22) Date of filing: 12.10.2011
(51) Int. Cl.: A61B 17/28, A61B 17/29

(54) **TREATMENT APPARATUS**
BEHANDLUNGSWERKZEUG
OUTIL DE TRAITEMENT

(30) Priority: 01.11.2010 JP 2010245492
(43) Date of publication of application: 13.03.2013
(73) Proprietor: OLYMPUS MEDICAL SYSTEMS CORP., Tokyo 151-0072 (JP)
(72) Inventor: NAITO, Kimihiko, Hachioji-shi Tokyo 192-8512 (JP)
(74) Representative: Schicker, Silvia
(86) International application number: PCT/JP2011/073420
(87) International publication number: WO 2012/060189

(56) References cited:
- JP-A- 2005 034 623
- JP-A- 2007 509 698
- US-A1- 2005 004 432
- US-A1- 2007 255 289
- US-A1- 2008 004 656

## Description

### Technical Field

The present invention relates to a treatment apparatus such as forceps or a manipulator which is configured to be inserted into the body cavity of a patient to treat a morbid section.

### Background Art

Patent Literature 1 discloses a high-frequency treatment apparatus which is configured to grip a morbid section with a grip portion including high-frequency electrodes to treat the morbid section. This high-frequency treatment apparatus includes an insertion section configured to be inserted into a body cavity, and an operation section provided to a proximal direction side of the insertion section. The insertion section includes a distal treatment section provided with a grip portion, and a flexible tube section which is provided to the proximal direction side of the distal treatment section and which extends in longitudinal directions. When a rotating operation of the distal treatment section is performed, the rotational torque generated by the rotating operation of the operation section is transferred to the distal treatment section via a conductive line extending through the flexible tube section. This rotates the distal treatment section to directions about an axis relative to the flexible tube section. In addition, pulling or loosening the conductive line will make the grip portion perform opening/closing movement between an open state and a closed state. That is, the conductive line serves as a rotating operation transfer member which transfers rotating operation to the distal treatment section and a grip operation transfer member which transfers grip operation to the grip portion. Patent literature 2 discloses a treatment apparatus having the features of the preamble of claim 1.

### Citation List

### Patent Literature

Patent Literature 1: Jpn. Pat. Appln. KOKAI Publication No. 2009-142513. Patent Literature 2: US-A-2005/0004432.

### Summary of Invention

### Technical Problem

Since the high-frequency treatment apparatus in Patent Literature 1 is configured such that the conductive line, which transfers the rotational torque generated at the operation section, extends in longitudinal directions, the rotational torque to the proximal direction side of the conductive line may not be properly transferred to the distal treatment section. In this case, the rotation follow-up of the distal treatment section deteriorates. As a result, the rotation amount of the operation section differs from that of the distal treatment section or a rotating movement of the distal treatment section fluctuates.

There is also conceivable a treatment apparatus which is provided with a pair of rotating operation wires, in addition to the conductive line as a grip operation transfer member, to rotate a distal treatment section relative to a flexible tube section. In this case, the treatment apparatus includes a rotor which is provided between the flexible tube section and the distal treatment section, and which is configured to rotate together with the distal treatment section relative to the flexible tube section. Each rotating operation wire is fixed to the rotor or a portion to a distal direction side of the rotor, and extends on the outer surface of the rotor along an oblique direction inclining from the longitudinal directions to the circumferential directions. A direction changing portion, provided to a portion to a proximal direction side of the rotor, is configured to change the extending direction of each rotating operation wire from the oblique direction. Each rotating operation wire, whose extending direction has been changed by the direction changing portion, extends to the rotating operation section provided to the proximal direction side of the flexible tube section. When rotating the distal treatment section, each rotating operation wire is pulled or loosened by performing rotating operation with the rotating operation section. This makes the distal treatment section and the rotor rotate relative to the flexible tube section.

However, when the distal treatment section rotates relative to the flexible tube section, torsion occurs in the grip operation transfer member to be connected to the grip portion of the distal treatment section. When the torsion of the grip operation transfer member is increased by increasing the amount of rotation of the distal treatment section, a force preventing the torsion acts on the distal treatment section. The force preventing the torsion acts in a direction opposite to the rotation direction of the distal treatment section, and hence leads to a deterioration in operability in rotating operation. When a large torque acts on the distal treatment section as, for example, a morbid section is gripped with the grip portion, in particular, the force preventing the torsion of the grip operation transfer member increases.

The present invention has been developed in view of the above circumstances, and an object thereof is to provide a treatment apparatus which exhibits high operability in the rotating operation of rotating a distal treatment section without causing any torsion in a grip operation transfer member.

### Solution to Problem

To solve above mentioned problems, according to the invention, a treatment apparatus includes a flexible tube section which extends in longitudinal directions, and which has a longitudinal directions axis; a distal treatment section which includes a grip portion configured to perform opening/closing movement between an open state and a closed state, and which is provided to a distal direction side of the flexible tube section so as to be rotatable to the directions about an axis relative to the flexible tube section; a grip operation section which is provided to a proximal direction side of the flexible tube section, and which is configured to perform grip operation of the grip portion; a grip operation linear portion which extends from the grip operation section into the flexible tube section and, which is configured to be moved along the longitudinal directions axis by the grip operation with the grip operation section; and further features as claimed in claim 1.

Preferred embodiments are disclosed in the dependent claims.

### Advantageous Effects of Invention

According to the present invention, a treatment apparatus, which exhibits high operability in the rotating operation of rotating a distal treatment section without causing any torsion in a grip operation transfer member, can be provided.

### Brief Description of Drawings

FIG. 1 is a schematic view showing a treatment apparatus according to the first embodiment of the present invention;
FIG. 2 is a sectional view schematically showing the configuration of the operation section of the treatment apparatus according to the first embodiment;
FIG. 3 is a sectional view taken along a line III - III in FIG. 1;
FIG. 4 is a perspective view schematically showing the configuration of a distal direction side part of the treatment apparatus according to the first embodiment;
FIG. 5 is a sectional view schematically showing the configuration of the distal direction side part of the treatment apparatus according to the first embodiment;
FIG. 6 is s sectional view schematically showing the distal direction side part of the treatment apparatus according to the first embodiment using a section different from that of FIG. 5;
FIG. 7 is a perspective view schematically showing the configuration of the distal direction side part of the treatment apparatus according to the first embodiment with the outer cover being removed;
FIG. 8 is a bottom view schematically showing the configuration of the distal direction side part of the treatment apparatus according to the first embodiment with the outer cover being removed;
FIG. 9 is an enlarged sectional view of a range X1 in FIG. 5;
FIG. 10 is an enlarged sectional view of a range X2 in FIG. 6;
FIG. 11 is a sectional view schematically showing the distal direction side part of the treatment apparatus according to the first embodiment while the actuated member is in the second operation state;
FIG. 12 is a schematic view explaining a rotating movement of the distal treatment section of the treatment apparatus according to the first embodiment;
FIG. 13 is a schematic view showing a treatment apparatus according to the first modification of the first embodiment;
FIG. 14 is a perspective view schematically showing the distal direction side part of a treatment apparatus according to the second modification of the first embodiment;
FIG. 15 is a sectional view schematically showing the configuration between the grip operation wire and the grip portion of a treatment apparatus according to the third modification of the first embodiment;
FIG. 16 is a sectional view schematically showing the configuration between the grip operation wire and the grip portion of a treatment apparatus according to the fourth modification of the first embodiment;
FIG. 17 is a sectional view schematically showing the configuration between the grip operation wire and the grip portion of a treatment apparatus according to the fifth modification of the first embodiment;
FIG. 18 is a sectional view schematically showing the configuration of the distal direction side part of a treatment apparatus according to the sixth modification of the first embodiment;
FIG. 19 is a sectional view schematically showing the configuration of the distal direction side part of a treatment apparatus according to the second embodiment of the present invention;
FIG. 20 is a sectional view schematically showing the configuration of the distal direction side part of a treatment apparatus according to the third embodiment of the present invention;
FIG. 21 is a sectional view taken along a line 21 - 21 in FIG. 20; and
FIG. 22 is a sectional view schematically showing an example of the interior of the flexible tube section of the treatment apparatus according to the third embodiment while the shape of the flexible tube section has changed.

### Description of Embodiments

### (First Embodiment)

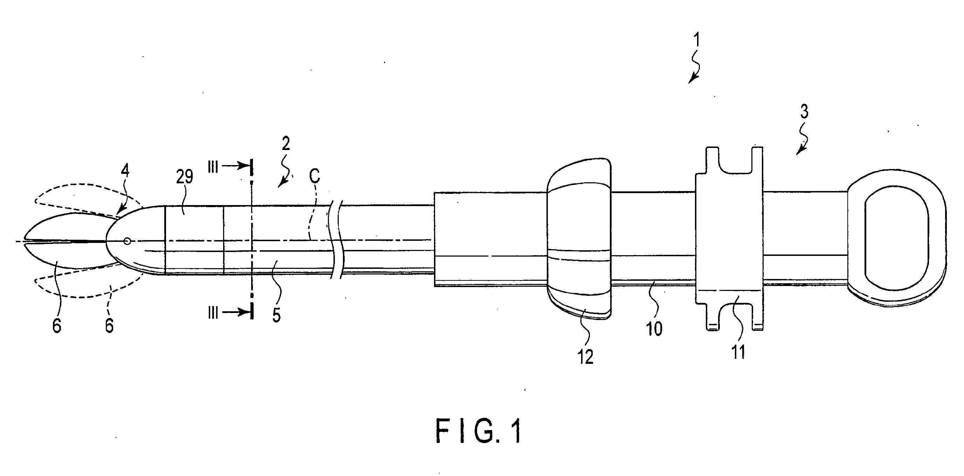

The first embodiment of the present invention will be described with reference to FIGS. 1 to 12.

FIG. 1 is a view showing the configuration of a treatment apparatus 1 according to this embodiment. As shown in FIG. 1, the treatment apparatus 1 includes an insertion section 2 configured to be inserted into a body cavity, and an operation section 3 provided to a proximal direction side to the insertion section 2. The insertion section 2 includes a distal treatment section 4 which is configured to perform treatment, and a flexible tube section 5 which is provided to the proximal direction side of the distal treatment section 4 and extends in longitudinal directions. The distal treatment section 4 is provided with a grip portion 6 which is configured to grip a tissue or the like. The grip portion 6 is configured to perform opening/closing movement between an open state (indicated by the dotted lines in FIG. 1) and a closed state (indicated by the solid lines in FIG. 1). The flexible tube section 5 has a longitudinal directions axis C.

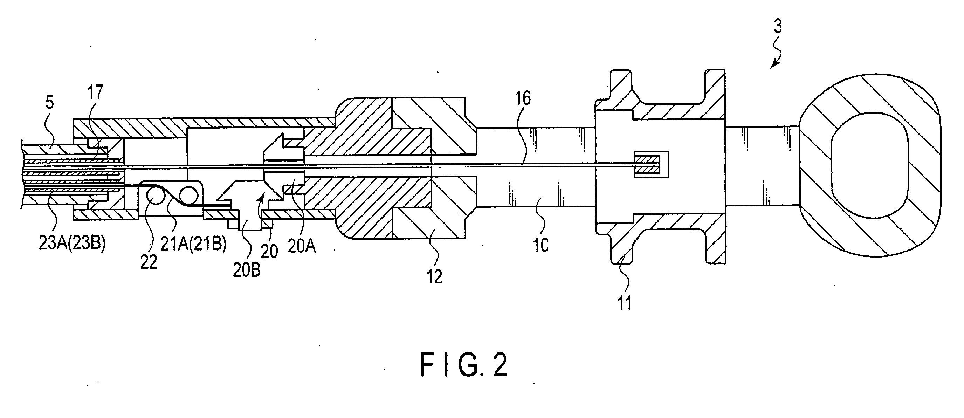

FIG. 2 is a view showing the configuration of the operation section 3. As shown in FIG. 2, the operation section 3 includes an operation section main body 10, a grip operation handle 11 serving as a grip operation section which is configured to perform the grip operation of gripping a tissue or the like with the grip portion 6, and a rotating operation handle 12 serving as a rotating operation section which is configured to perform the rotating operation of rotating the distal treatment section 4 to directions about an axis relative to the flexible tube section 5.

The grip operation handle 11 is mounted on the operation section main body 10 so as to be movable in the longitudinal directions relative to the operation section main body 10. A proximal end of a grip operation wire 16 serving as a grip operation transfer member, which transfers grip operation to the grip portion 6, is fixed to the grip operation handle 11. The grip operation wire 16 extends in the longitudinal directions through the interior of the flexible tube section 5. Inside the flexible tube section 5, the grip operation wire 16 extends through a coil pipe (third coil pipe) 17 used in grip operation. The grip operation wire 16 is pulled or loosened by moving the grip operation handle 11 in the longitudinal directions relative to the operation section main body 10.

The rotating operation handle 12 is mounted on the operation section main body 10 so as to be rotatable to the directions about (around) the axis relative to the operation section main body 10. A bevel gear 20 is coupled to the rotating operation handle 12. The bevel gear 20 includes a first gear 20A coupled to the rotating operation handle 12, and a second gear 20B meshed with the first gear 20A. Proximal ends of first and second rotating operation wires 21A and 21B serving as rotating operation transfer members, which transfer rotating operation to the distal treatment section 4, are connected to the second gear 20B. The first and second rotating operation wires 21A and 21B are guided into the flexible tube section 5 by guide pulleys 22, and extend in almost longitudinal directions inside the flexible tube section 5. Inside the flexible tube section 5, the first rotating operation wire 21A extends through a coil pipe (first coil pipe) 23A used in rotating operation, and the second rotating operation wire 21B extends through a coil pipe (second coil pipe) 23B used in rotating operation. When the rotating operation handle 12 is rotated to the directions about the axis, the first gear 20A of the bevel gear 20 rotates together with the rotating operation handle 12 to the directions about the axis. As the first gear 20A rotates, the second gear 20B rotates about an axis perpendicular to the longitudinal directions. As the second gear 20B rotates in one rotation direction, the first rotating operation wire 21A is pulled, and the second rotating operation wire 21B is loosened. As the second gear 20B rotates in the other rotation direction, the first rotating operation wire 21A is loosened, and the second rotating operation wire 21B is pulled.

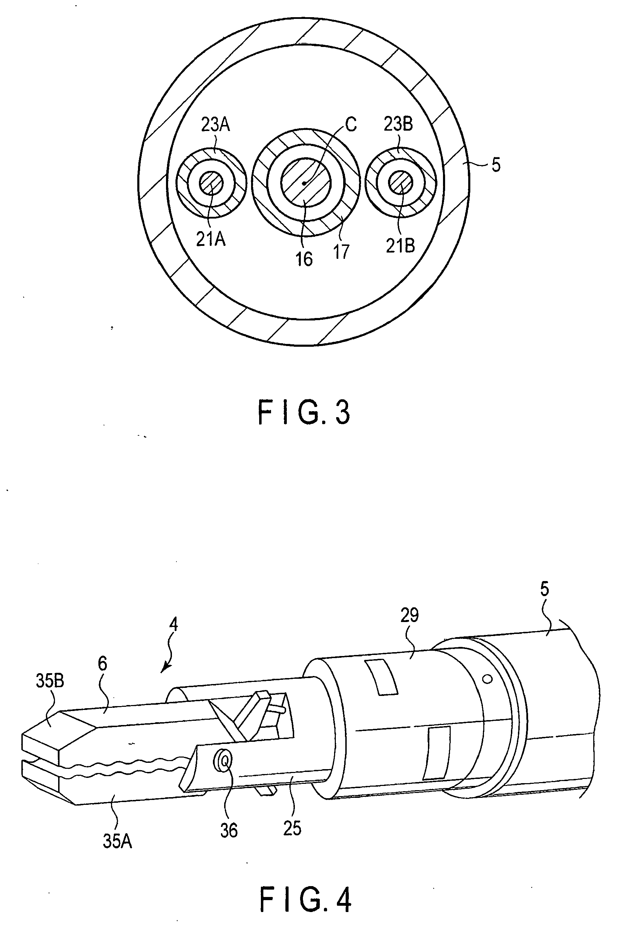

FIG. 3 is a sectional view taken along a line III - III in FIG. 1. As shown in FIG. 3, inside the flexible tube section 5, the grip operation wire 16 extends in a state that its axis almost coincides with the longitudinal directions axis C of the flexible tube section 5. In addition, inside the flexible tube section 5, the first and second rotating operation wires 21A and 21B are provided to an outer direction side of an outer surface of the coil pipe 17. The first rotating operation wire 21A is spaced apart from the second rotating operation wire 21B by almost 180° in the directions about the axis. With this configuration, an inner diameter of the flexible tube section 5 is larger than the sum of outer diameters of the coil pipe 17 and coil pipes 23A and 23B.

FIGS. 4 to 6 are views showing the configuration of the distal direction side part of the treatment apparatus 1. As shown in FIGS. 4 to 6, a rotor 26 is provided between the distal treatment section 4 and the flexible tube section 5 while being fixed to the distal treatment section 4. A rotor support member 27 is provided between the rotor 26 and the flexible tube section 5. The rotor support member 27 is fixed to the flexible tube section 5. The rotor 26 is coupled to the rotor support member 27 so as to be rotatable to the directions about the axis. A cylindrical outer cover 29 is provided to the outer direction side of the rotor support member 27. The outer cover 29 is fixedly coupled to the flexible tube section 5. This configuration allows the distal treatment section 4 and the rotor 26 to rotate together to the directions about the axis relative to the flexible tube section 5, the rotor support member 27, and the outer cover 29.

As shown in FIGS. 4 to 6, the distal treatment section 4 includes a treatment section main body 25. A first clamping portion 35A and a second clamping portion 35B, which constitute the grip portion 6, are pivotably supported on the treatment section main body 25 through a coupling pin 36. The first and second clamping portions 35A and 35B can rotate together with the treatment section main body 25 to the directions about the axis relative to the flexible tube section 5. In addition, the first and second clamping portions 35A and 35B can rotate about the coupling pin 36 relative to the treatment section main body 25. When the first and second clamping portions 35A and 35B rotate relative to the treatment section main body 25, the grip portion 6 performs opening/closing movement between the closed state and the open state.

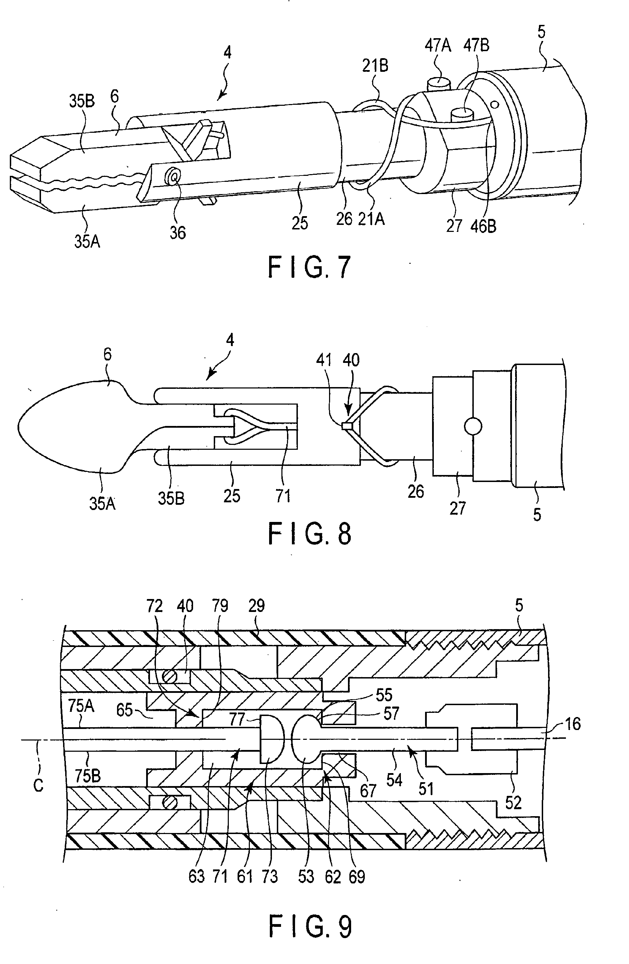

FIGS. 7 and 8 are views showing the configuration of the distal direction side part of the treatment apparatus 1 with the outer cover 29 being removed. As shown in FIG. 8, a proximal direction side part of the treatment section main body 25 (distal treatment section 4) is provided with a through hole 41. The coupling portion between the rotor 26 and the distal treatment section 4 is provided with a wire fixing portion 40 which fixes the distal ends of the first and second rotating operation wires 21A and 21B. The first rotating operation wire 21A, whose distal end is fixed to the wire fixing portion 40, extends to the outer direction side of the treatment section main body 25 through the through hole 41. Likewise, the second rotating operation wire 21B, whose distal end is fixed to the wire fixing portion 40, extends to the outer direction side of the treatment section main body 25 through the through hole 41.

In this embodiment, the rotor 26 is provided with the wire fixing portion 40. However, the embodiment is not limited to this. For example, the treatment section main body 25 of the distal treatment section 4 may be provided with the wire fixing portion 40 to which the distal ends of the first and second rotating operation wires 21A and 21B are fixed. That is, the wire fixing portion 40 is provided to the rotor 26 or a portion to the distal direction side of the rotor 26. In addition, although the distal treatment section 4 and the rotor 26 are separate members, they may be integrally formed. That is, the distal treatment section 4 and the rotor 26 may be configured to rotate to the direction about the axis relative to the flexible tube section 5.

As shown in FIG. 7, an outer surface of the rotor support member 27 is provided with a first convex portion 47A and a second convex portion 47B which protrude in the outer direction. The first convex portion 47A is spaced apart from the through hole 41 in the circumferential directions. The second convex portion 47B is spaced apart from the through hole 41 in the circumferential directions toward a direction opposite to a direction toward the first convex portion 47A. A first hole portion 46A and a second hole portion 46B are provided to a portion to the proximal direction side of the first convex portion 47A (second convex portion 47B) of the rotor support member 27.

As shown in FIGS. 7 and 8, the first rotating operation wire 21A, extending from the wire fixing portion 40 in the outer direction, extends on the outer surface of the rotor 26 along a first oblique direction inclining from the longitudinal directions to the circumferential directions. The first rotating operation wire 21A, extending on the outer surface of the rotor 26, abuts against the first convex portion 47A of the rotor support member 27. Making the first rotating operation wire 21A abut against the first convex portion 47A will change an extending direction of the first rotating operation wire 21A from the first oblique direction. The first rotating operation wire 21A is then inserted into the flexible tube section 5 from the first hole portion 46A. The first rotating operation wire 21A, inserted in the flexible tube section 5, extends to the rotating operation section (rotating operation handle 12).

The second rotating operation wire 21B, extending from the wire fixing portion 40 in the outer direction, extends on the outer surface of the rotor 26 along a second oblique direction inclining from the longitudinal directions to the circumferential directions toward a direction opposite to a direction toward the first oblique direction. The second rotating operation wire 21B, extending on the outer surface of the rotor, abuts against the second convex portion 47B of the rotor support member 27. Making the second rotating operation wire 21B abut against the second convex portion 47B will change an extending direction of the second rotating operation wire 21B from the second oblique direction. The second rotating operation wire 21B is then inserted from the second hole portion 46B into the flexible tube section 5. The second rotating operation wire 21B, inserted in the flexible tube section 5, extends to the rotating operation section (rotating operation handle 12).

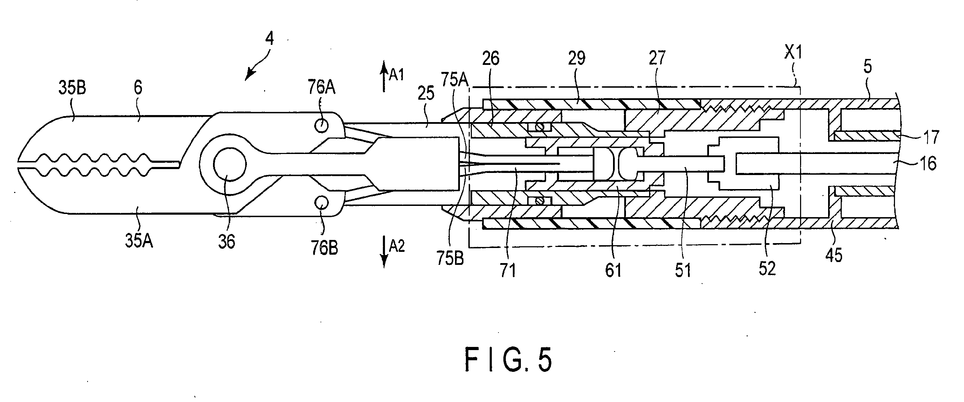

As shown in FIG. 5, a distal end portion of the flexible tube section 5 is provided with a coil lock 45. A distal end of the coil pipe 17 used in grip operation, through which the grip operation wire 16 extends, is fixed to the coil lock 45. The grip operation wire 16 extends to the distal direction side of the distal end of the coil pipe 17.

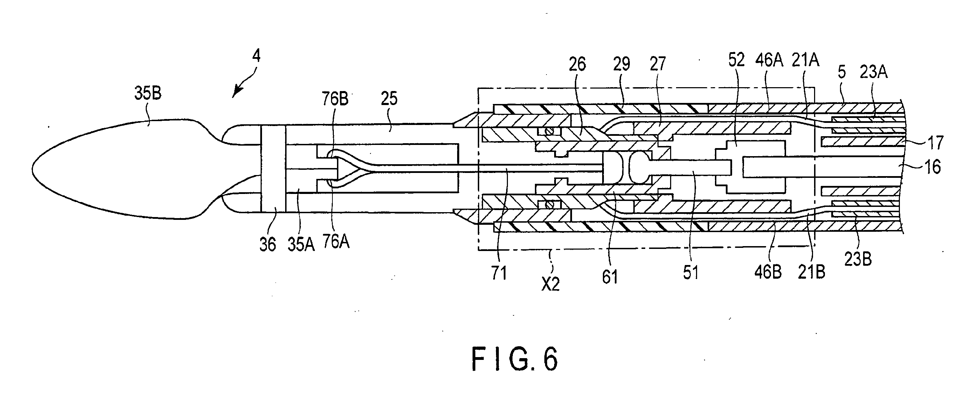

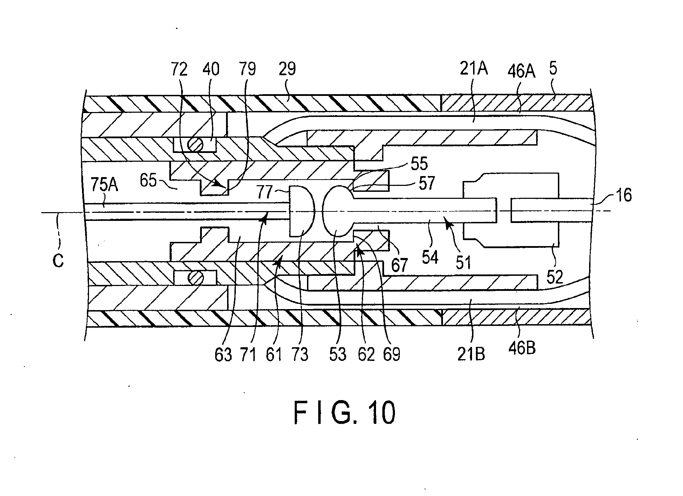

FIG. 9 is an enlarged view of a range X1 in FIG. 5. FIG. 10 is an enlarged view of a range X2 in FIG. 6. As shown in FIGS. 9 and 10, a coupling shaft 51 as a wire distal member is provided to the distal direction side of the grip operation wire 16. The coupling shaft 51 is provided in a state that the coupling shaft 51 is fixed to the grip operation wire 16 through a junction member 52. When the grip operation wire 16 is pulled or loosened, the coupling shaft 51 moves in the longitudinal directions. The coupling shaft 51 includes a large-diameter portion 53, and a small-diameter portion 54 continuous to the proximal direction side of the large-diameter portion 53. A step portion 55 is provided between the large-diameter portion 53 and the small-diameter portion 54. The step portion 55 includes a curved surface 57 having an arcuated section taken parallel to the longitudinal directions axis C.

A shaft bearing 61 as a coupling member is fixed to the inner surface of the rotor 26. The shaft bearing 61 can rotate together with the distal treatment section 4 and the rotor 26 to the directions about the axis relative to the coupling shaft 51. The shaft bearing 61 includes a first coupling portion 62 with which the coupling shaft 51 is coupled to the shaft bearing 61 so as to be movable in the longitudinal directions. An accommodation portion 63, in Which the large-diameter portion 53 of the coupling shaft 51 is accommodated, is formed in the shaft bearing 61. The accommodation portion 63 is provided with an opening portion 65 which is open toward the distal direction. A hole-like portion 67 is formed to the proximal direction side of the accommodation portion 63 so as to communicate with the accommodation portion 63. The small-diameter portion 54 of the coupling shaft 51 extends through the hole-like portion 67. The small-diameter portion 54 of the coupling shaft 51 is coupled to the junction member 52 at a portion to the proximal direction side of a proximal end of the shaft bearing 61.

A proximal direction side part of the shaft bearing 61 is provided with a first regulation portion 69 which is configured to regulate an amount of movement of the coupling shaft 51 in the proximal direction. The first regulation portion 69 is formed by providing a step on the inner surface of the shaft bearing 61. Making the step portion 55 of the coupling shaft 51 abut against the first regulation portion 69 will prevent the movement of the coupling shaft 51 in the proximal direction. That is, the step portion 55 serves as the first abutment portion, which is configured to prevent the movement of the coupling shaft 51 in the proximal direction by abutting against the first regulation portion 69. The step portion 55 abuts against the first regulation portion 69 of the shaft bearing 61 on the curved surface 57. In the above manner, with the first coupling portion 62, the coupling shaft 51 is coupled to the shaft bearing 61 so as to be movable in the longitudinal directions.

An actuated member 71 is provided to the distal direction side of the coupling shaft 51 while being connected to the grip portion 6. The shaft bearing 61 includes a second coupling portion 72 to which the actuated member 71 is coupled so as to be rotatable together with the distal treatment section 4 to the directions about the axis relative to the coupling shaft 51 and the flexible tube section 5. In addition, the actuated member 71 is coupled to the shaft bearing 61 so as to be movable in the longitudinal directions. The actuated member 71 includes a columnar portion 73, and first and second rod-like portions 75A and 75B extending from the columnar portion 73 in the distal direction. The first rod-like portion 75A is connected to the first clamping portion 35A through a first connecting portion 76A, and the second rod-like portion 75B is connected to the second clamping portion 35B through a second connecting portion 76B (see FIG. 5 and 6). A step portion 77 is provided between the columnar portion 73 and the first rod-like portion 75A (second rod-like portion 75B).

The shaft bearing 61 is provided with a second regulation portion 79 which is configured to regulate an amount of movement of the actuated member 71 in the distal direction. The second regulation portion 79 is formed by providing a step on the inner surface of the shaft bearing 61. Making the step portion 77 of the actuated member 71 abut against the second regulation portion 79 will prevent the movement of the actuated member 71 in the distal direction. That is, the step portion 77 serves as the second abutment portion, which is configured to prevent the movement of the actuated member 71 in the distal direction by abutting against the second regulation portion 79. In the above manner, the actuated member 71 is coupled to the second coupling portion 72 of the shaft bearing 61 so as to be rotatable to the directions about the axis relative to the coupling shaft 51. The actuated member 71 is a moving member coupled to the second coupling portion 72 of the shaft bearing 61 so as to be movable in the longitudinal directions relative to the shaft bearing 61. In addition, the shaft bearing 61 is a rotating member coupled to the coupling shaft 51 at the first coupling portion 62 so as to be rotatable together with the actuated member 71 to the directions about the axis relative to the coupling shaft 51.

An actuated state of the actuated member 71 changes in accordance with the movement of the coupling shaft 51 in the longitudinal directions. The actuated state of the actuated member 71 changes between the first actuated state in which the grip portion 6 is actuated to the closed state (indicated by the solid lines in FIG. 1) and the second actuated state in which the grip portion 6 is actuated to the open state (indicated by the dotted lines in FIG. 1).

FIG. 5 is a view showing the distal direction side part of the treatment apparatus 1 when the actuated member 71 is in the first actuated state. In the first actuated state, the actuated member 71 is not in contact with the coupling shaft 51, and hence no force acts from the coupling shaft 51. When no force acts from the coupling shaft 51, the actuated member 71 changes to the first actuated state due to, for example, the biasing force of a biasing member (not shown). In addition, the first rod-like portion 75A is biased by, for example, an elastic force to a state in which it opens in a direction of an arrow A1 in FIG. 5. Likewise the second rod-like portion 75B is biased by, for example, an elastic force to a state in which it opens in a direction of an arrow A2 in FIG. 5. In the first actuated state, the inner surface of the shaft bearing 61 regulates the opening movement of the first and second rod-like portions 75A and 75B. Regulating the opening movement of the first and second rod-like portions 75A and 75B prevents the first and second clamping portions 35A and 35B from pivoting from the closed state relative to the treatment section main body 25. Therefore, when the actuated member 71 is in the first actuated state, the grip portion 6 is actuated to the closed state.

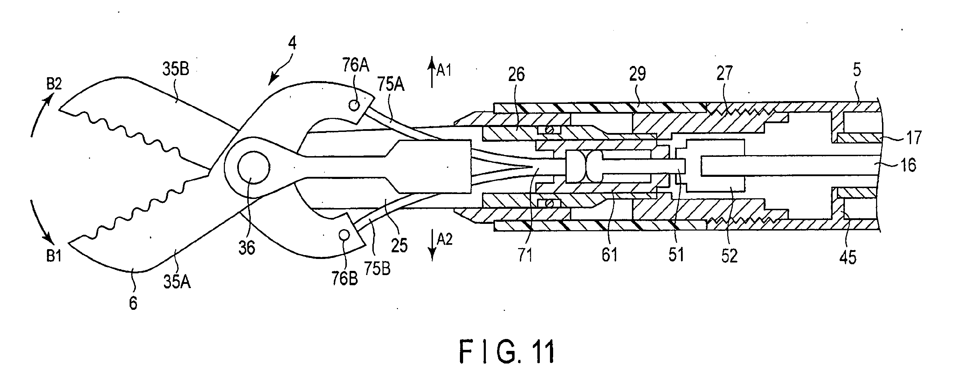

FIG. 11 is a view showing the distal direction side part of the treatment apparatus 1 when the actuated member 71 is in the second actuated state. When the grip operation wire 16 is loosened by gripping operation with the grip operation handle 11, the coupling shaft 51 as a wire distal member moves in the distal direction. When the coupling shaft 51 moves in the distal direction while the actuated member 71 is in the first actuated state, the coupling shaft 51 comes into contact with the actuated member 71. As a consequence, a force acts from the coupling shaft 51 to the actuated member 71. This causes the actuated member 71 to move in the distal direction and changes the actuated state to the second actuated state. That is, when the actuated member 71 moves from the first actuated state in the distal direction in accordance with the movement of the coupling shaft 51 in the distal direction, the actuated state changes to the second actuated state. In the second actuated state, the inner surface of the shaft bearing 61 does not regulate the opening movement of the first and second rod-like portions 75A and 75B. For this reason, the first rod-like portion 75A opens in the direction of an arrow A1 in FIG. 11, and the second rod-like portion 75B opens in the direction of an arrow A2 in FIG. 11. When the first rod-like portion 75A opens, the first clamping portion 35A pivots in a direction of an arrow B1 in FIG. 11 relative to the treatment section main body 25. When the second rod-like portion 75B opens, the second clamping portion 35B pivots in a direction of an arrow B2 in FIG. 11 relative to the treatment section main body 25. When, therefore, the actuated member 71 is in the second actuated state, the grip portion 6 is actuated to the open state.

When the grip operation wire 16 is pulled by performing grip operation with the grip operation handle 11, the coupling shaft 51 as a wire distal member moves in the proximal direction. When the coupling shaft 51 moves in the proximal direction while the actuated member 71 is in the second actuated state, no force acts from the coupling shaft 51 to the actuated member 71. As described above, when no force acts from the coupling shaft 51, the actuated member 71 moves in the proximal direction, and the actuated member 71 changes to the first actuated state. That is, when the actuated member 71 moves in the proximal direction from the second actuated state in accordance with the movement of the coupling shaft 51 in the proximal direction, the actuated state changes to the first actuated state. When the actuated member 71 changes to the first actuated state, the grip portion 6 is actuated to the closed state.

The function of the treatment apparatus 1 according to this embodiment will be described next. When rotating the distal treatment section 4 of the treatment apparatus 1 to the directions about the axis relative to the flexible tube section 5, the rotating operation handle 12 is rotated in one rotation direction. This pulls the first rotating operation wire 21A and loosens the second rotating operation wire 21B through the bevel gear 20. When the first rotating operation wire 21A is pulled, the distal treatment section 4 rotates in the first rotation direction which is one rotation direction.

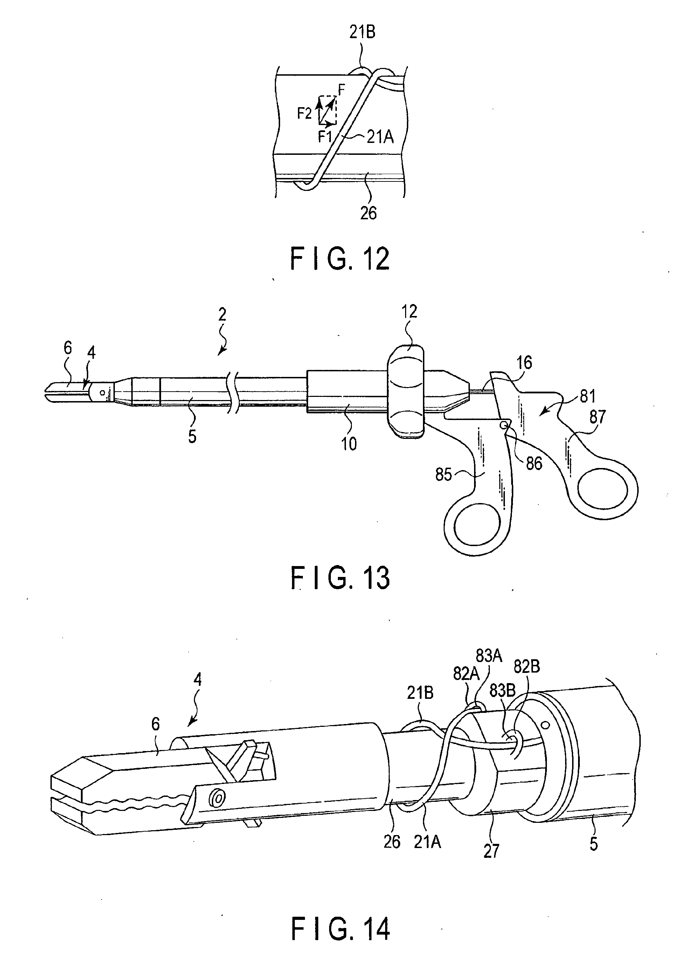

FIG. 12 is a view explaining the rotating movement of the distal treatment section 4. The first rotating operation wire 21A extends on the outer surface of the rotor 26, between the wire fixing portion 40 and the first convex portion 47A, along the first oblique direction inclining from the longitudinal directions to the circumferential directions. In this case, the first oblique direction is a direction inclining from the longitudinal directions to the first rotation direction. As shown in FIG. 12, when the first rotating operation wire 21A is pulled, a force F acts on the rotor 26 in the first oblique direction. The force F is decomposed into a force F1 in the longitudinal directions and a rotational force F2 in the first rotation direction (circumferential directions). The rotational force F2 rotates the rotor 26 in the first rotation direction. At this time, the distal treatment section 4 rotates together with the rotor 26 to the directions about the axis. In the above manner, the distal treatment section 4 and the rotor 26 rotate in the first rotation direction relative to the flexible tube section 5 and the rotor support member 27.

On the other hand, when the rotating operation handle 12 is rotated in the other rotation direction, the first rotating operation wire 21A is loosened and the second rotating operation wire 21B is pulled through the bevel gear 20. Pulling the second rotating operation wire 21B will rotate the distal treatment section 4 in the second rotation direction opposite to the first rotation direction. The second rotating operation wire 21B extends on the outer surface of the rotor 26, between the wire fixing portion 40 and the second convex portion 47B, along the second oblique direction inclining from the longitudinal directions to the circumferential directions. In this case, the second oblique direction is a direction inclining from the longitudinal directions to the second rotation direction. When the second rotating operation wire 21B is pulled, a force acts on the rotor 26 in the second oblique direction. The force in the second oblique direction is decomposed into a force in the longitudinal directions and a rotational force in the second rotation direction (circumferential directions). With the rotational force in the second rotation direction, the rotor 26 rotates in the second rotation direction. At this time, the distal treatment section 4 rotates together with rotor 26 to the directions about the axis. In the above manner, the distal treatment section 4 and the rotor 26 rotate in the second rotation direction relative to the flexible tube section 5 and the rotor support member 27.

When gripping a tissue or the like with the grip portion 6 of the distal treatment section 4, the grip operation handle 11 is moved in the longitudinal directions relative to the operation section main body 10 to pull or loosen the grip operation wire 16. When no force acts from the coupling shaft 51, the actuated member 71 changes to the first actuated state. In the first actuated state, the inner surface of the shaft bearing 61 regulates the opening movement of the first and second rod-like portions 75A and 75B. Since the opening movement of the first and second rod-like portions 75A and 75B is regulated, the first and second clamping portions 35A and 35B do not pivot from the closed state relative to the treatment section main body 25. As a consequence, the grip portion 6 is actuated to the closed state.

When the grip operation wire 16 is loosened by performing griping operation with the grip operation handle 11, the coupling shaft 51 as a wire distal member moves in the distal direction. When the coupling shaft 51 moves in the distal direction while the actuated member 71 is in the first actuated state, the coupling shaft 51 comes into contact with the actuated member 71, and a force acts from the coupling shaft 51 onto the actuated member 71 in the distal direction. With this operation, the actuated member 71 moves in the distal direction, and the actuated state changes to the second actuated state. In the second actuated state, the inner surface of the shaft bearing 61 does not regulate the opening movement of the first and second rod-like portions 75A and 75B. For this reason, the opening movement of the first rod-like portion 75A makes the first clamping portion 35A pivot relative to the treatment section main body 25 in the direction of the arrow B1 in FIG. 11, and the opening movement of the second rod-like portion 75B makes the second clamping portion 35B pivot relative to the treatment section main body 25 in the direction of the arrow B2 in FIG. 11. As a consequence, the grip portion 6 is actuated to the open state.

When the grip operation wire 16 is pulled by performing griping operation with the grip operation handle 11, the coupling shaft 51 as a wire distal member moves in the proximal direction. When the coupling shaft 51 moves in the proximal direction while the actuated member 71 is in the second actuated state, no force acts from the coupling shaft 51 onto the actuated member 71. As described above, when no force acts from the coupling shaft 51, the actuated member 71 moves in the proximal direction and changes to the first actuated state. When the actuated member 71 changes to the first actuated state, the grip portion 6 is actuated to the closed state. The grip portion 6 then grips a tissue or the like in the closed state.

The treatment apparatus 1 is provided with the coupling shaft 51 as a wire distal member which is fixed to the grip operation wire 16. The coupling shaft 51 is coupled to the first coupling portion 62 of the shaft bearing 61 as a coupling member so as to be movable in the longitudinal directions relative to the shaft bearing 61. In addition, the actuated member 71 is coupled to the second coupling portion 72 of the shaft bearing 61 so as to be rotatable together with the distal treatment section 4 to the directions about the axis relative to the coupling shaft 51 and the flexible tube section 5. Since the actuated member 71 is rotatable together with the distal treatment section 4 relative to the coupling shaft 51, no torsion occurs in the grip operation wire 16 when the distal treatment section 4 rotates relative to the flexible tube section 5.

The first coupling portion 62 of the shaft bearing 61 is provided with the first regulation portion 69 which is configured to regulate the amount of movement of the coupling shaft 51 in the proximal direction. The coupling shaft 51 includes the step portion 55 (first abutment portion) which is configured to prevent the movement of the coupling shaft 51 in the proximal direction by abutting against the first regulation portion 69. Providing the first regulation portion 69 and the step portion 55 couples the coupling shaft 51 to the first coupling portion 62 while the coupling shaft 51 is movable in the longitudinal directions relative to the shaft bearing 61. The second coupling portion 72 of the shaft bearing 61 is provided with the second regulation portion 79 which is configured to regulate the amount of movement of the actuated member 71 in the distal direction. The actuated member 71 includes the step portion 77 (second abutment portion) which is configured to prevent the movement of the actuated member 71 in the distal direction by abutting against the second regulation portion 79. Providing the second regulation portion 79 and the step portion 77 couples the actuated member 71 to the second coupling portion 72 while the actuated member 71 is movable in the longitudinal directions relative to the shaft bearing 61.

The shaft bearing 61 is a rotating member which is coupled to the coupling shaft 51 at the first coupling portion 62 so as to be rotatable together with the actuated member 71 to the directions about the axis relative to the coupling shaft 51. The step portion 55 of the coupling shaft 51 includes the curved surface 57 which abuts against the first regulation portion 69 of the shaft bearing 61. Since the curved surface 57 of the coupling shaft 51 abuts against the first regulation portion 69, the contact area is small when the step portion 55 abuts against the first regulation portion 69. For this reason, even when the shaft bearing 61 rotates together with the distal treatment section 4 to the directions about the axis relative to the coupling shaft 51 while the step portion 55 is in contact with the first regulation portion 69, the friction between the step portion 55 and the first regulation portion 69 is small.

The treatment apparatus 1 having the above arrangement therefore has the following effects. The treatment apparatus 1 according to this embodiment is provided with the coupling shaft 51 serving as a wire distal member fixed to the grip operation wire 16. When the grip operation wire 16 is pulled or loosened, the coupling shaft 51 moves in the longitudinal directions. As the coupling shaft 51 moves in the longitudinal directions, the actuated state of the actuated member 71 changes between the first actuated state in which the grip portion 6 is actuated to the closed state and the second actuated state in which the grip portion 6 is actuated to the open state. Changing the actuated state of the actuated member 71 makes the grip portion 6 perform opening/closing movement to grip a tissue or the like. In this case, the coupling shaft 51 is coupled to the first coupling portion 62 of the shaft bearing 61 serving as a coupling member so as to be movable in the longitudinal directions relative to the shaft bearing 61. In addition, the actuated member 71 is coupled to the second coupling portion 72 of the shaft bearing 61 so as to be rotatable together with the distal treatment section 4 to the directions about the axis relative to the coupling shaft 51 and the flexible tube section 5. Since the actuated member 71 is rotatable together with the distal treatment section 4 relative to the coupling shaft 51, no torsion occurs in the grip operation wire 16 even when the distal treatment section 4 rotates relative to the flexible tube section 5. In the above manner, it is possible to provide the treatment apparatus 1 which exhibits high operability in the rotating operation of rotating the distal treatment section 4 without causing any torsion in the grip operation wire 16 serving as a grip operation transfer member.

The first coupling portion 62 of the shaft bearing 61 is provided with the first regulation portion 69 which is configured to regulate the amount of movement of the coupling shaft 51 in the proximal direction. The coupling shaft 51 includes the step portion 55 (first abutment portion) which is configured to prevent the movement of the coupling shaft 51 in the proximal direction by abutting against the first regulation portion 69. Providing the first regulation portion 69 and the step portion 55 can couple the coupling shaft 51 to the first coupling portion 62 so as to allow the coupling shaft 51 to move in the longitudinal directions relative to the shaft bearing 61. In addition, the second coupling portion 72 of the shaft bearing 61 is provided with the second regulation portion 79 which is configured to regulate the amount of movement of the actuated member 71 in the distal direction. The actuated member 71 includes the step portion 77 (second abutment portion) which is configured to prevent the movement of the actuated member 71 in the distal direction by abutting against the second regulation portion 79. Providing the second regulation portion 79 and the step portion 77 can couple the actuated member 71 to the second coupling portion 72 so as to allow the actuated member 71 to move in the longitudinal directions relative to the shaft bearing 61.

The shaft bearing 61 is a rotating member which is coupled to the coupling shaft 51 at the first coupling portion 62 so as to be rotatable together with the actuated member 71 to the directions about the axis relative to the coupling shaft 51. The step portion 55 of the coupling shaft 51 includes the curved surface 57 which abuts against the first regulation portion 69 of the shaft bearing 61. Since the curved surface 57 of the coupling shaft 51 abuts against the first regulation portion 69, the contact area is small when the step portion 55 abuts against the first regulation portion 69. For this reason, even when the shaft bearing 61 rotates together with the distal treatment section 4 to the directions about the axis relative to the coupling shaft 51 while the step portion 55 is in contact with the first regulation portion 69, the friction between the step portion 55 and the first regulation portion 69 is small. Therefore, when the distal treatment section 4 rotates relative to the flexible tube section 5 while the step portion 55 is in contact with the first regulation portion 69, it is possible to prevent a deterioration in operability in rotating operation.

### (Modification of First Embodiment)

A modification of the first embodiment will be described with reference to FIGS. 13 to 18. The same reference numerals as in the first embodiment denote the same parts and parts having the same functions, and a description of them will be omitted.

FIG. 13 is a view showing the configuration of the treatment apparatus 1 according to the first modification of the first embodiment. As shown in FIG. 13, the operation section 3 of the treatment apparatus 1 is provided with a grip operation section 81 which is configured to perform the grip operation of gripping a tissue or the like with the grip portion 6. The grip operation section 81 includes a fixed handle 85 fixed to the operation section main body 10, and a movable handle 87 which is pivotably supported on the fixed handle 85 through a connecting pin 86. The movable handle 87 can pivot about the connecting pin 86 relative to the fixed handle 85. A proximal end of the grip operation wire 16 is connected to the movable handle 87. Pivoting the movable handle 87 in a direction to close relative to the fixed handle 85 will pull the grip operation wire 16. Pivoting the movable handle 87 in a direction to open relative to the fixed handle 85 will loosen the grip operation wire 16.

As described above, as indicated by the first modification, the configuration of pulling or loosening the grip operation wire 16 is not limited to that in the embodiment described above. Likewise, the configuration of pulling or loosening the first and second rotating operation wires 21A and 21B is not limited to that in the embodiment described above.

FIG. 14 is a view showing the distal direction side part of the treatment apparatus 1 according to the second modification of the first embodiment. As shown in FIG. 14, the rotor support member 27 in this modification is not provided with the first and second convex portions 47A and 47B. Instead of these parts, the rotor support member 27 is provided with a first guide portion 82A and a second guide portion 82B. The first guide portion 82A includes a first insertion hole 83A. The second guide portion 82B includes a second insertion hole 83B.

As shown in FIG. 14, the first rotating operation wire 21A, extending from the wire fixing portion 40, extends on the outer surface of the rotor 26 along the first oblique direction inclining from the longitudinal directions to the first rotation direction. The first rotating operation wire 21A is then inserted into the first insertion hole 83A of the first guide portion 82A. Inserting the first rotating operation wire 21A into the first insertion hole 83A of the first guide portion 82A will change the extending direction of the first rotating operation wire 21A from the first oblique direction. The first rotating operation wire 21A, whose extending direction has been changed from the first oblique direction by the first guide portion 82A, is inserted from the first hole portion 46A into the flexible tube section 5. The first rotating operation wire 21A, which has been inserted into the flexible tube section 5, extends to the rotating operation section (rotating operation handle 12).

On the other hand, the second rotating operation wire 21B, extending from the wire fixing portion 40, extends on the outer surface of the rotor 26 along the second oblique direction inclining from the longitudinal directions to the second rotation direction. The second rotating operation wire 21B is then inserted into the second insertion hole 83B of the second guide portion 82B. Inserting the second rotating operation wire 21B into the second insertion hole 83B of the second guide portion 82B will change the extending direction of the second rotating operation wire 21B from the second oblique direction. The second rotating operation wire 21B, whose extending direction has been changed from the second oblique direction by the second guide portion 82B, is inserted from the second hole portion 46B into the flexible tube section 5. The second rotating operation wire 21B, which has been inserted into the flexible tube section 5, extends to the rotating operation section (rotating operation handle 12).

As described above, according to the second modification, the configuration of changing the extending direction of the first rotating operation wire 21A from the first oblique direction and the configuration of changing the extending direction of the second rotating operation wire 21B from the second oblique direction are not limited to those in the embodiment described above. That is, this apparatus may be provided with a direction changing portion which is configured to make the first rotating operation wire 21A extend to the rotating operation section by changing, from the first oblique direction, the extending direction of the first rotating operation wire 21A, which has extended from the wire fixing portion 40 onto the outer surface of the rotor 26 along the first oblique direction. Likewise, the apparatus may be provided with a direction changing portion which is configured to make the second rotating operation wire 21B extend to the rotating operation section by changing, from the second oblique direction, the extending direction of the second rotating operation wire 21B, which has extended from the wire fixing portion 40 onto the outer surface of the rotor 26 along the second oblique direction.

In addition, the first and second convex portions 47A and 47B may be provided to the distal direction side part of the flexible tube section 5. That is, the first and second convex portions 47A and 47B may be provided to the distal direction side part of the flexible tube section 5 or another member such as the rotor support member 27 provided between the flexible tube section 5 and the distal treatment section 4 while being fixed to the flexible tube section 5.

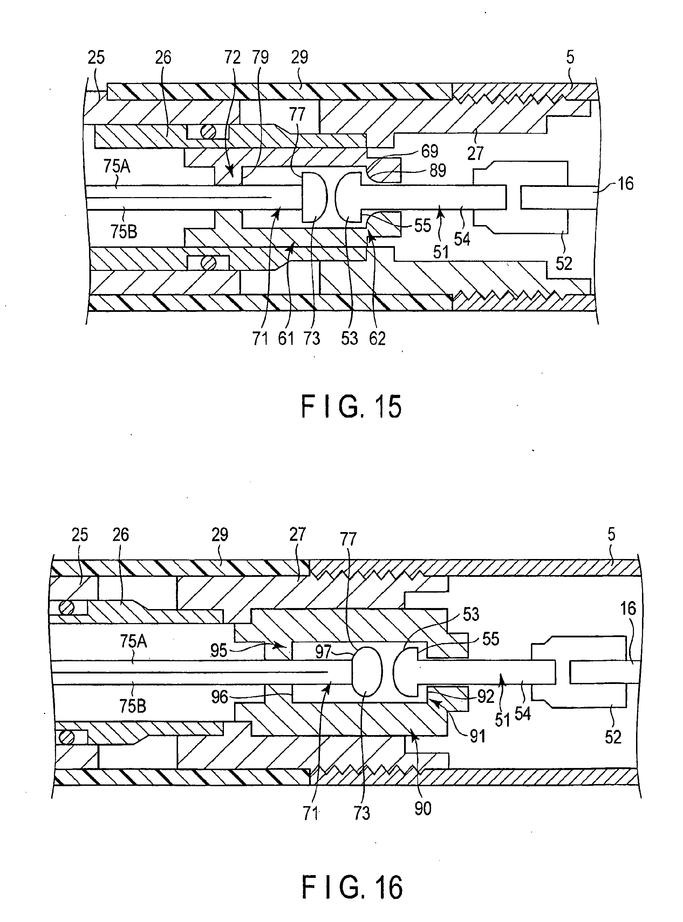

FIG. 15 is a view showing an configuration between the grip operation wire 16 and the grip portion 6 according to the third modification of the first embodiment. As shown in FIG. 15, in this modification, as in the first embodiment, the coupling shaft 51 as a wire distal member is provided in a state that the coupling shaft 51 is fixed to the grip operation wire 16. The coupling shaft 51 is coupled to the first coupling portion 62 of the shaft bearing 61 so as to be movable in the longitudinal directions relative to the shaft bearing 61. The actuated member 71, to be connected to the grip portion 6, is coupled to the second coupling portion 72 of the shaft bearing 61. The actuated member 71 can rotate together with the shaft bearing 61 to the directions about the axis relative to the coupling shaft 51. The first coupling portion 62 of the shaft bearing 61 is provided with the first regulation portion 69 which is configured to regulate the amount of movement of the coupling shaft 51 in the proximal direction. The coupling shaft 51 includes the step portion 55 which is configured to prevent the movement of the coupling shaft 51 in the proximal direction by abutting against the first regulation portion 69.

In this modification, instead of the step portion 55, the first regulation portion 69 of the shaft bearing 61 is provided with a curved surface 89, having an arcuated section taken parallel to the longitudinal directions axis C of the flexible tube section 5, which abuts against the step portion 55 of the coupling shaft 51. Since the step portion 55 abuts against the curved surface 89 of the first regulation portion 69, the contact area is small when the step portion 55 abuts against the first regulation portion 69. For this reason, even when the shaft bearing 61 rotates together with the distal treatment section 4 to the directions about the axis relative to the coupling shaft 51 in a state that the step portion 55 is in contact with the first regulation portion 69, the friction between the step portion 55 and the first regulation portion 69 is small.

As described above, according to the third modification, it is possible to use any configuration in which the contact area is small when the step portion 55 abuts against the first regulation portion 69. With such an configuration, even when the shaft bearing 61 rotates together with the distal treatment section 4 to the directions about the axis relative to the coupling shaft 51 while the step portion 55 is in contact with the first regulation portion 69, the friction between the step portion 55 and the first regulation portion 69 is small. Therefore, when the distal treatment section 4 rotates relative to the flexible tube section 5 while the step portion 55 is in contact with the first regulation portion 69, it is possible to prevent a deterioration in operability in rotating operation.

FIG. 16 is a view showing the configuration between the grip operation wire 16 and the grip portion 6 according to the fourth modification of the first embodiment. As shown in FIG. 16, in this modification, as in the first embodiment, the coupling shaft 51, which is a wire distal member, is provided so as to be fixed to the grip operation wire 16. In this modification, the inner surface of the rotor 26 is not provided with the shaft bearing 61. Instead of this part, the inner surface of the rotor support member 27 is provided with a shaft bearing 90. That is, the shaft bearing 90 is a fixing member provided so as to be fixed to the flexible tube section 5.

The coupling shaft 51 is fixed to a first coupling portion 91 of the shaft bearing 90 so as to be movable in the longitudinal directions relative to the shaft bearing 90. The first coupling portion 91 of the shaft bearing 90 is provided with a first regulation portion 92 which is configured to regulate the amount of movement of the coupling shaft 51 in the proximal direction. The coupling shaft 51 includes the step portion 55 which is configured to prevent the movement of the coupling shaft 51 in the proximal direction by abutting against the first regulation portion 92. In addition, the actuated member 71, to be connected to the grip portion 6, is coupled to a second coupling portion 95 of the shaft bearing 90. The actuated member 71 is coupled to the second coupling portion 95 of the shaft bearing 61 so as to be rotatable to the directions about the axis relative to the shaft bearing 90. The second coupling portion 95 of the shaft bearing 90 is provided with a second regulation portion 96 which is configured to regulate the amount of movement of the actuated member 71 in the distal direction. The actuated member 71 includes the step portion 77 which is configured to prevent the movement of the actuated member 71 in the distal direction by abutting against the second regulation portion 96.

In this modification, the step portion (second abutment portion) 77 of the actuated member 71 is provided with a curved surface 97, having an arcuated section taken parallel to the longitudinal directions axis C of the flexible tube section 5, which abuts against the second regulation portion 96 of the shaft bearing 90. Since the curved surface 97 of the step portion 77 abuts against the second regulation portion 96, the contact area is small when the step portion 77 abuts against the second regulation portion 96. For this reason, even when the actuated member 71 rotates together with the distal treatment section 4 to the directions about the axis relative to the shaft bearing 90 and the coupling shaft 51 while the step portion 77 is in contact with the second regulation portion 96, the friction between the step portion 77 and the second regulation portion 96 is small.

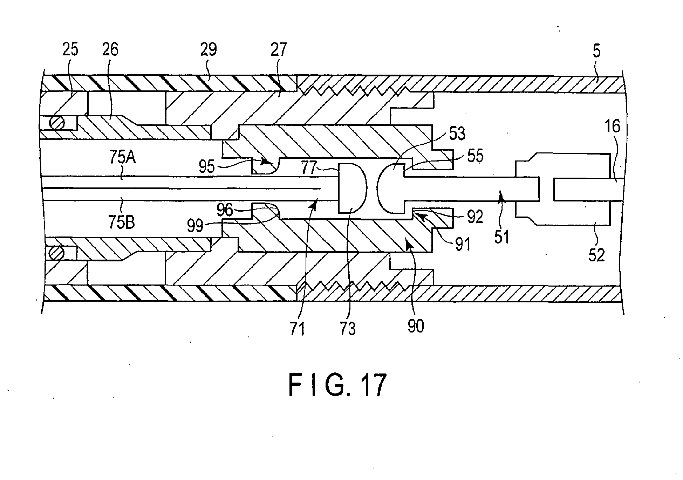

FIG. 17 is a view showing the configuration between the grip operation wire 16 and the grip portion 6 according to the fifth modification of the first embodiment. As shown in FIG. 17, in this modification, as in the fourth modification of the first embodiment, the coupling shaft 51, which is a wire distal member, is provided in a state that the coupling shaft 51 is fixed to the grip operation wire 16. The coupling shaft 51 is coupled to the first coupling portion 91 of the shaft bearing 90 so as to be movable in the longitudinal directions relative to the shaft bearing 90. In addition, the actuated member 71, to be connected to the grip portion 6, is coupled to the second coupling portion 95 of the shaft bearing 90. The actuated member 71 is rotatable to the directions about the axis relative to the shaft bearing 90 and the coupling shaft 51. The second coupling portion 95 of the shaft bearing 90 is provided with the second regulation portion 96 which is configured to regulate the amount of movement of the actuated member 71 in the distal direction. The actuated member 71 includes the step portion 77 which is configured prevent the movement of the actuated member 71 in the distal direction by abutting against the second regulation portion 96.

In this modification, instead of the step portion 77, the second regulation portion 96 of the shaft bearing 90 is provided with a curved surface 99, having an arcuated section taken parallel to the longitudinal directions axis C of the flexible tube section 5, which abuts against the step portion 77 of the actuated member 71. Since the step portion 77 abuts against the curved surface 99 of the second regulation portion 96, the contact area is small when the step portion 77 abuts against the second regulation portion 96. For this reason, even when the actuated member 71 rotates together with the distal treatment section 4 to the directions about the axis relative to the coupling shaft 51 and the shaft bearing 90 while the step portion 77 is in contact with the second regulation portion 96, the friction between the step portion 77 and the second regulation portion 96 is small.

As described above, according to the fourth and fifth modifications, this apparatus may be provided with the shaft bearing 90, which is a fixing member, to which the actuated member 71 is coupled at the second coupling portion 95 so as to be rotatable to the directions about the axis, instead of the shaft bearing 61, which is a rotating member, which is coupled to the coupling shaft 51 at the first coupling portion 62 so as to be rotatable together with the actuated member 71 to the directions about the axis relative to the coupling shaft 51. In this case, the shaft bearing 90 is provided in a state that the shaft bearing 90 is fixed to the flexible tube section 5. In this case, it is possible to use any configuration in which the contact area is small when the step portion 77 abuts against the second regulation portion 96. With such an configuration, even when the actuated member 71 rotates together with the distal treatment section 4 to the directions about the axis relative to the coupling shaft 51 and the shaft bearing 90 while the step portion 77 is in contact with the second regulation portion 96, the friction between the step portion 77 and the second regulation portion 96 is small. Therefore, when the distal treatment section 4 rotates relative to the flexible tube section 5 while the step portion 77 is in contact with the second regulation portion 96, it is possible to prevent a deterioration in operability in rotating operation.

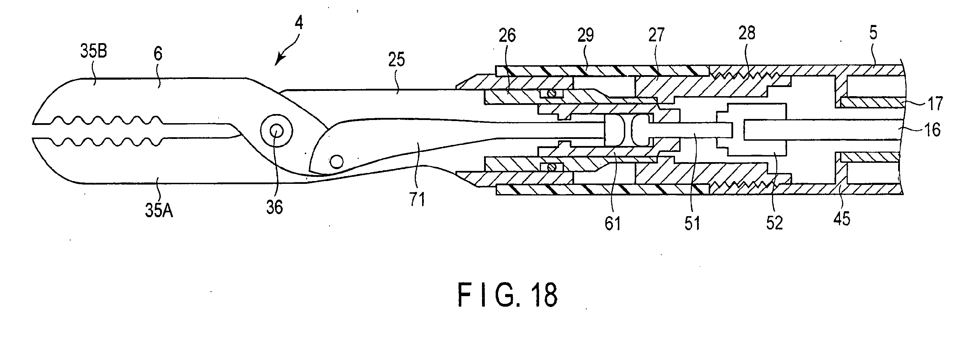

FIG. 18 is a view showing the configuration of the distal direction side part of the treatment apparatus 1 according to the sixth modification of the first embodiment. As shown in FIG. 18, the first clamping portion 35A of the grip portion 6 is provided in a state that the first clamping portion 35A is fixed to the treatment section main body 25. In addition, the second clamping portion 35B of the grip portion 6 is pivotably supported on the treatment section main body 25 through the coupling pin 36. The second clamping portion 35B can rotate together with the treatment section main body 25 to the directions about the axis relative to the flexible tube section 5. The second clamping portion 35B can pivot about the coupling pin 36 relative to the treatment section main body 25. When the second clamping portion 35B pivots relative to the treatment section main body 25, the second clamping portion 35B performs opening/closing movement relative to the first clamping portion 35A of the treatment section main body 25.

The actuated member 71 is connected to the second clamping portion 35B. When the grip operation wire 16 is pulled or loosened with the grip operation handle 11, the actuated member 71 moves in the longitudinal directions in accordance with pulling or loosening of the grip operation wire 16, as described above. As the actuated member 71 moves, the second clamping portion 35B pivots about the coupling pin 36 relative to the treatment section main body 25. As the actuated member 71 moves in the distal direction, the second clamping portion 35B pivots in a direction to open relative to the first clamping portion 35A. On the other hand, when the actuated member 71 moves in the proximal direction, the second clamping portion 35B pivots in a direction to close relative to the first clamping portion 35A.

As described above, according to the sixth modification, the configuration of the grip portion 6 is not limited to that in the above embodiment. That is, the distal treatment section 4 may include the grip portion 6 which is configured to perform opening/closing movement between the closed state and the open state.

### (Second Embodiment)

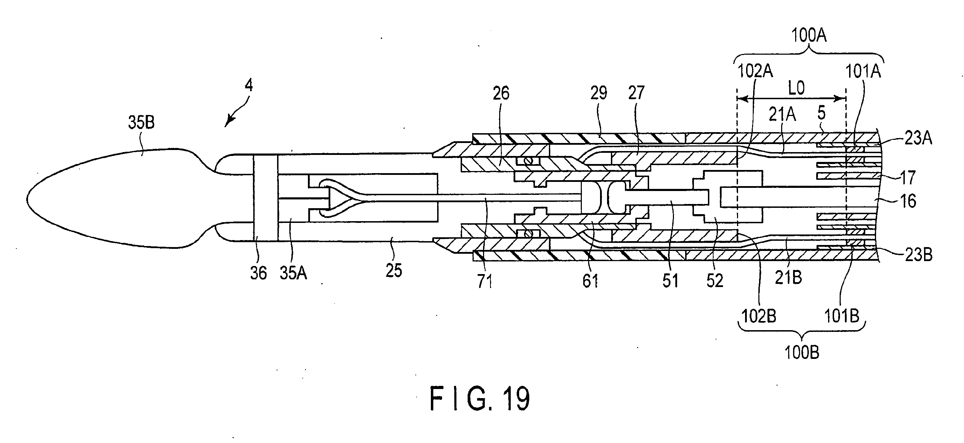

The second embodiment of the present invention will be described next with reference to FIG. 19. The same reference numerals as in the first embodiment denote the same parts and parts having the same functions, and a description of them will be omitted.

FIG. 19 is a view showing the configuration of the distal direction side part of the treatment apparatus 1 according to the second embodiment. As shown in FIG. 19, in this embodiment, an outer surface of a first rotating operation wire 21A is provided with a first stopper member 101A in a fixed state. When the first rotating operation wire 21A is pulled or loosened by rotating a rotating operation handle 12, the first stopper member 101A moves in the longitudinal directions. Likewise, an outer surface of a second rotating operation wire 21B is provided with a second stopper member 101B in a fixed state. When the second rotating operation wire 21B is pulled or loosened by rotating a rotating operation handle 12, the second stopper member 101B moves in the longitudinal directions.

A rotor support member 27 is provided with a first bearing portion 102A which is configured to prevent the movement of the first stopper member 101A in the distal direction by making the first stopper member 101A abut against the first bearing portion 102A. Likewise, the rotor support member 27 is provided with a second bearing portion 102B which is configured to prevent the movement of the second bearing portion 102B in the distal direction by making the second stopper member 101B abut against the second bearing portion 102B.

While the distal treatment section 4 and a rotor 26 are in a neutral state, in which they are not rotating to the directions about the axis relative to a flexible tube section 5, the first stopper member 101A is spaced apart from the first bearing portion 102A by a predetermined distance L0 in the proximal direction. When the first stopper member 101A moves from the neutral state by the distance L0 in the distal direction, the first stopper member 101A abuts against the first bearing portion 102A. This prevents the movement of the first stopper member 101A in the distal direction. This regulates an amount of loosening of the first rotating operation wire 21A. Regulating the amount of loosening of the first rotating operation wire 21A will regulate an amount of pulling of the second rotating operation wire 21B. That is, the first stopper member 101A and the first bearing portion 102A constitute a first regulation portion 100A which is configured to regulate the amount of pulling of the second rotating operation wire 21B.

In the neutral state, the second stopper member 101B is spaced apart from the second bearing portion 102B by a predetermined distance L0 in the proximal direction. When the second stopper member 101B moves from the neutral state by the distance L0 in the distal direction, it abuts against the second bearing portion 102B. This prevents the movement of the second stopper member 101B in the distal direction. This regulates an amount of loosening of the second rotating operation wire 21B. Regulating the amount of loosening of the second rotating operation wire 21B will regulate an amount of pulling of the first rotating operation wire 21A. That is, the second stopper member 101B and the second bearing portion 102B constitute a second regulation member 100B which is configured to regulate the amount of pulling of the first rotating operation wire 21A.

The treatment apparatus 1 having the above arrangement therefore has the following effects in addition to the same effects as those of the first embodiment. That is, in the treatment apparatus 1 according to this embodiment, the first stopper member 101A and the first bearing portion 102A are configured to regulate the amount of pulling of the second rotating operation wire 21B. Likewise, the second stopper member 101B and the second bearing portion 102B are configured to regulate the amount of pulling of the first rotating operation wire 21A. Regulating the amount of pulling of the first and second rotating operation wires 21A and 21B will regulate the amount of rotation of a distal treatment section 4 and the rotor 26. This prevents excessive rotation of the distal treatment section 4, and hence can prevent the wire from being cut at a wire fixing portion 40.

Note that in the above embodiment, the first bearing portion 102A and the second bearing portion 102B are provided on the rotor support member 27. However, the embodiment is not limited to this. That is, the first bearing portion 102A and the second bearing portion 102B may be provided to the distal direction side of the flexible tube section 5 in a fixed state relative to the flexible tube section 5. It is therefore not preferable to provide the first bearing portion 102A and the second bearing portion 102B in the flexible tube section 5. If the first bearing portion 102A and the second bearing portion 102B are provided in the flexible tube section 5, a change in the shape of the flexible tube section 5 greatly influences the regulation of the amount of pulling of the first and second rotating operation wires 21A and 21B. It is also not preferable to provide the first and second bearing portions 102A and 102B to the rotor 26. Since the first and second rotating operation wires 21A and 21B extend on the outer surface of the rotor 26, the first and second bearing portions 102A and 102B are formed so as to protrude on the outer surface of the rotor 26 in the outer direction. This increases a diameter of the rotor 26.

### (Third Embodiment)

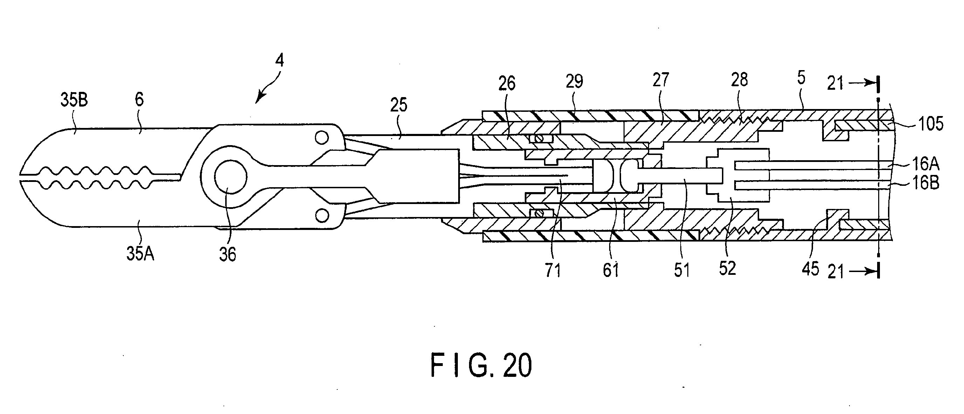

The third embodiment of the present invention will be described next with reference to FIGS. 20 to 22. The same reference numerals as in the first embodiment denote the same parts and parts having the same functions, and a description of them will be omitted.

FIG. 20 is a view showing the configuration of the distal direction side part of the treatment apparatus 1 according to the third embodiment. As shown in FIG. 20, in this embodiment, as in the first embodiment, inside a flexible tube section 5, a first rotating operation wire 21A extends through a coil pipe (first coil pipe) 23A used in rotating operation. A second rotating operation wire 21B extends through a coil pipe (second coil pipe) 23B used in rotating operation. The embodiment is also provided with a first grip operation wire 16A and a second grip operation wire 16B. The first and second grip operation wires 16A and 16B are pulled or loosened together by operation with a grip operation handle 11. Pulling or loosening the first and second grip operation wires 16A and 16B will move a coupling shaft 51 as a wire distal member in the longitudinal directions.

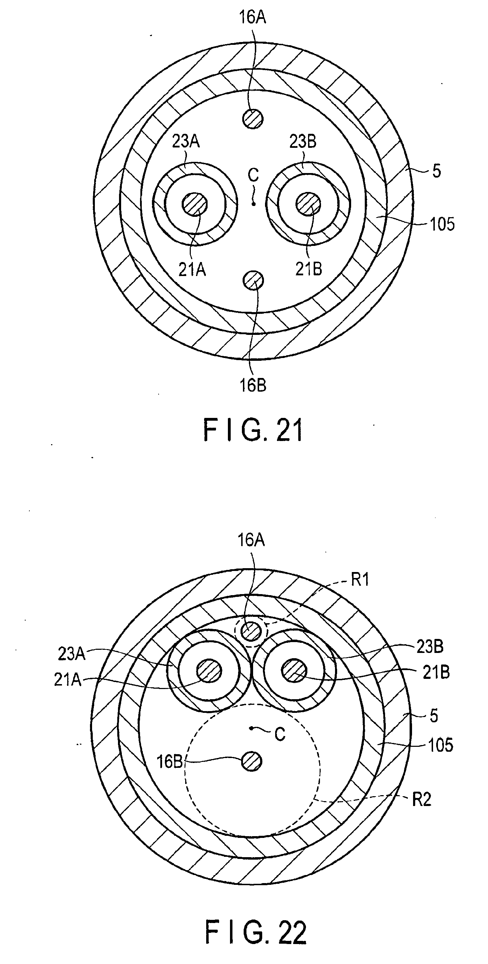

FIG. 21 is a sectional view taken along a line 21 - 21 in FIG. 20. As shown in FIG. 21, inside the flexible tube section 5, the first rotating operation wire 21A is spaced apart from the second rotating operation wire 21B by almost 180° in the directions about the axis. The first grip operation wire 16A extends spaced apart (by almost 90° in the directions about the axis in this embodiment) from the coil pipe 23A and the coil pipe 23B in the directions about axis. In addition, the second grip operation wire 16B extends space apart by almost 180° from the first grip operation wire 16A in the directions about the axis. In addition, the flexible tube section 5 is internally provided with a coil pipe (third coil pipe) 105 through which the first grip operation wire 16A, the second grip operation wire 16B, the coil pipe 23A, and the coil pipe 23B extend. In the first embodiment, the inner diameter of the flexible tube section 5 needs to be larger than the sum of the outer diameter of the coil pipe 17 and the outer diameters of the coil pipes 23A and 23B. In contrast to this, in the third embodiment, the inner diameter of the flexible tube section 5 may be larger than the sum of the outer diameters of the coil pipes 23A and 23B and a wall thickness of the coil pipe 105.

FIG. 22 is a view showing an example of the interior of the flexible tube section 5 while the shape of the flexible tube section 5 has changed. In the state shown in FIG. 22, with a change in the shape of the flexible tube section 5, the coil pipes 23A and 23B and the first and second grip operation wires 16A and 16B have moved. The coil pipes 23A and 23B are inscribed in the coil pipe 105, and the coil pipes 23A and 23B are circumscribed to each other. In this state, assume that a circle R1 is a reference circle, which is one of two circles R1 and R2 circumscribed around the coil pipes 23A and 23B and inscribed in the coil pipe 105, and which has a smaller diameter in the two circles R1 and R2. The diameter of the first grip operation wire 16A is smaller than that of the reference circle R1. Likewise, the diameter of the second grip operation wire 16B is smaller than that of the reference circle R1. With this configuration, the contact areas between the first grip operation wire 16A and the coil pipes 23A, 23B, and 105 decrease regardless of how the coil pipes 23A and 23B and the first and second grip operation wires 16A and 16B move. With regard to the second grip operation wire 16B as well, the contact portions with the coil pipes 23A and 23B and 105 decrease.

The treatment apparatus 1 having the above configuration has the following effects in addition to the same effects as those of the first embodiment. That is, in the treatment apparatus 1 according to this embodiment, the inner diameter of the flexible tube section 5 may be larger than the sum of the outer diameters of the coil pipes 23A and 23B and the wall thickness of the coil pipe 105. For this reason, it is possible to decrease the diameter of the flexible tube section 5 as compared with the first embodiment (the inner diameter of the flexible tube section 5 needs to be larger than the sum of the outer diameters of the coil pipe 17 and coil pipes 23A and 23B).

In the treatment apparatus 1, the diameter of the first grip operation wire 16A is smaller than that of the reference circle R1. Likewise, the diameter of the second grip operation wire 16B is smaller than the diameter of the reference circle R1. This reduces the contact portions between the first grip operation wire 16A and the coils 23A, 23B, and 105 regardless of how the coil pipes 23A and 23B and the first and second grip operation wires 16A and 16B move. Likewise, this also reduces the contact portions between the second grip operation wire 16B and the coil pipes 23A and 23B and 105. This makes it possible to ensure the sliding property of the first and second grip operation wires 16A and 16B when they are pulled or loosened.

Incidentally, the present invention is not limited to the above embodiments and various modifications can naturally be made without deviating from the scope of the present invention, as claimed.

## Claims

1. A treatment apparatus (1) comprising:
a flexible tube section (5) which extends in longitudinal directions, and which has a longitudinal directions axis (C);
a distal treatment section (4) which includes a grip portion (6) configured to perform opening/closing movement between an open state and a closed state, and which is provided to a distal direction side of the flexible tube section (5) so as to be rotatable to the directions about an axis relative to the flexible tube section (5);
a grip operation section (11;81) which is provided to a proximal direction side of the flexible tube section (5), and which is configured to input grip operation of the grip portion (6);
a grip operation linear portion (16,51,52;16A,16B,51,52) which extends from the grip operation section (11;81) into the flexible tube section (5), and which is moved along the longitudinal directions axis (C) by the grip operation of the grip operation section (11;81), the grip operation linear portion (16,51,52;16A,16B,51,52) including a first small-diameter portion (54), and a first large-diameter portion (53) which is provided to the distal direction side of the first small-diameter portion (54) and which has a diameter larger than that of the first small-diameter portion (54);
an actuated member (71) which is provided to be connected to the grip portion (6), and whose actuated state is configured to change between a first actuated state in which the grip portion (6) is actuated to the closed state and a second actuated state in which the grip portion (6) is actuated to the open state,
**characterized in that** the actuated member (71) includes a second small-diameter portion (75A,75B), and a second large-diameter portion (73) which is provided to the proximal direction side of the second small-diameter portion (75A,75B) and which has a diameter larger than that of the second small-diameter portion (75A,75B); and
the treatment apparatus (1) further comprises a junction coupling member (61;90) which is provided between a distal end portion of the grip operation linear portion (16,51,52;16A,16B,51,52) and a proximal end portion of the actuated member (71), and which couples between the grip operation linear portion (16,51,52;16A,16B,51,52) and the actuated member (71) so as to change the actuated state of the actuated member (71) in accordance with movement of the grip operation linear portion (16,51,52;16A,16B,51,52) along the longitudinal directions axis and so as to allow the actuated member (71) to rotate the directions about the axis relative to the grip operation linear portion (16,51,52;16A,16B,51,52),
wherein the junction coupling member (61;90) includes a coupling member (61;90) in which an accommodation hollow (63) is formed, and
the coupling member (61;90) includes a movement range regulation portion (62,72;91,95) to which at least one of the first large-diameter portion (53) and the second large-diameter portion (73) is rotatably coupled, and which is configured to regulate a movement range of at least one of the first large-diameter portion (53) and the second large-diameter portion (73) in the longitudinal directions, the movement range regulation portion (62,72;91,95) protruding toward the accommodation hollow (63) in an inner direction.

2. The treatment apparatus (1) of claim 1, **characterized in that**
the grip operation linear portion (16,51,52;16A,16B,51,52) includes a grip operation wire (16;16A,16B) which is pulled or loosened by the grip operation with the grip operation section (11;81), and a wire distal member (51) which is provided to the distal direction side of the grip operation wire (16;16A,16B) so as to be fixed to the grip operation wire (16;16A,16B) and which is configured to move in the longitudinal directions upon pulling or loosening of the grip operation wire (16;16A,16B),
the actuated member (71) is a moving member (71) which is coupled to the movement range regulation portion (62,72;91,95) of the coupling member (61,90) so as to move in the longitudinal directions relative to the coupling member (61,90), and
the moving member (71) is configured to change in actuated state to the second actuated state when moving from the first actuated state in the distal direction in accordance with movement of the wire distal member (51) in the distal direction, and configured to change in actuated state to the first actuated state when moving from the second actuated state in the proximal direction in accordance with movement of the wire distal member (51) in the proximal direction.

3. The treatment apparatus (1) of claim 2, **characterized in that**
the movement range regulation portion (62,72;91,95) includes a first coupling portion (62;91) to which the wire distal member (51) is coupled so as to move in the longitudinal directions, and a second coupling portion (72;95) to which the actuated member (71) is coupled so as to rotate together with the distal treatment section (4) to the directions about the axis relative to the wire distal member (51) and the flexible tube section (5),
the first coupling portion (62;91) includes a first regulation portion (69;92) which is configured to regulate an amount of movement of the wire distal member (51) in the proximal direction,
the wire distal member (51) includes a first abutment portion (55) which is configured to prevent movement of the wire distal member (51) in the proximal direction by abutting against the first regulation portion (69;92),
the second coupling portion (72;95) includes a second regulation portion (79;96) which is configured to regulate an amount of movement of the moving member (71) in the distal direction, and
the moving member (71) includes a second abutment portion (77) which is configured to prevent movement of the moving member (71) in the distal direction by abutting against the second regulation portion (79;96).

4. The treatment apparatus (1) of claim 3, **characterized in that**
the coupling member (61;90) is a rotating member (61) which is coupled to the wire distal member (51) at the first coupling portion (62) so as to rotate together with the moving member (71) to the directions about the axis relative to the wire distal member (51), and
the first abutment portion (55) includes a curved surface (57) which is configured to abut against the first regulation portion (69), and which has an arcuated section taken parallel to the longitudinal directions axis (C) of the flexible tube section (5).

5. The treatment apparatus (1) of claim 3, **characterized in that**
the coupling member (61;90) is a rotating member (61) which is coupled to the wire distal member (51) at the first coupling portion (62) so as to rotate together with the moving member (71) to the directions about the axis relative to the wire distal member (51), and
the first regulation portion (69) includes a curved surface (89) against which the first abutment portion (55) is configured to abut, and which has an arcuated section taken parallel to the longitudinal directions axis (C) of the flexible tube section (5).

6. The treatment apparatus (1) of claim 3, **characterized in that**
the coupling member (61;90) is a fixing member (90) which is provided to be fixed to the flexible tube section (5), and to which the moving member (71) is coupled with the second coupling portion (95) so as to rotate to the directions about the axis, and
the second abutment portion (77) includes a curved surface (97) which is configured to abut against the second regulation portion (96), and which has an arcuated section taken parallel to the longitudinal directions axis (C) of the flexible tube section (5).

7. The treatment apparatus (1) of claim 3, **characterized in that**
the coupling member (61;90) is a fixing member (90) which is provided to be fixed to the flexible tube section (5), and to which the moving member (71) is coupled with the second coupling portion (95) so as to rotate to the directions about the axis, and
the second regulation portion (96) includes a curved surface (99) against which the second abutment portion (77) is configured to abut, and which has an arcuated section taken parallel to the longitudinal directions axis (C) of the flexible tube section (5).

8. The treatment apparatus (1) of claim 3, **characterized by** further comprising:
a rotor (26) which is provided between the distal treatment section (4) and the flexible tube section (5), and which is configured to rotate together with the distal treatment section (4) to the directions about the axis relative to the flexible tube section (5);
a rotating operation section (12) which is provided to the proximal direction side of the flexible tube section (5), and which is configured to perform rotating operation of the distal treatment section (4);
a rotating operation wire (21A,21B) which has a distal end fixed to the rotor (26) or to a portion to the distal direction side of the rotor (26), and which extends to the rotating operation section (12) through an outer surface of the rotor (26) and an interior of the flexible tube section (5), the rotating operation wire (21A,21B) being configured to rotate the distal treatment section (4) by being pulled or loosened by the rotating operation with the rotating operation section (12); and
a pulling amount regulation portion (100A,100B) configured to regulate an amount of pulling of the rotating operation wire (21A,21B).

9. The treatment apparatus (1) of claim 8, **characterized in that** the pulling amount regulation portion (100A,100B) includes
a stopper member (101A,101B) which is provided in the flexible tube section (5) to be fixed to the rotating operation wire (21A,21B), and which is configured to move in the longitudinal directions when the rotating operation wire (21A,21B) is pulled or loosened, and
a bearing portion (102A,102B) which is provided to the distal direction side of the flexible tube section (5) to be fixed to the flexible tube section (5), and which is configured to prevent movement of the stopper member (101A,101B) in the distal direction by abutment of the stopper member (101A,101B).

## Patentansprüche

1. Behandlungsapparat (1) umfassend:
einen flexiblen Röhrenabschnitt (5), der sich in Längsrichtungen erstreckt und der eine Achse (C) in den Längsrichtungen hat;
einen distalen Behandlungsabschnitt (4), der einen Griffabschnitt (6) umfasst, welcher dazu eingerichtet ist, eine Öffnungs/Schließ-Bewegung zwischen einem offenen Zustand und einem geschlossenen Zustand auszuführen und welcher an einer Seite einer distalen Richtung des flexiblen Röhrenabschnitts (5) vorgesehen ist, um um eine Achse relativ zu dem flexiblen Röhrenabschnitt (5) in die Richtungen drehbar zu sein;
einen Griffbetätigungsabschnitt (11; 81), der an einer Seite einer proximalen Richtung des flexiblen Röhrenabschnitts (5) vorgesehen ist und der dazu eingerichtet ist, eine Griffbetätigung des Griffabschnitts (6) einzugeben;
einen linearen Griffbetätigungsabschnitt (16, 51, 52; 16A, 16B, 51, 52), der sich von dem Griffbetätigungsabschnitt (11; 81) in den flexiblen Röhrenabschnitt (5) erstreckt und der durch die Griffbetätigung des Griffbetätigungsabschnitts (11; 81) entlang der Achse (C) in den Längsrichtungen bewegt wird, wobei der lineare Griffbetätigungsabschnitt (16, 51, 52; 16A, 16B, 51, 52) einen ersten Abschnitt (54) mit einem kleinen Durchmesser und einen ersten Abschnitt (53) mit einem großen Durchmesser umfasst, der an der Seite der distalen Richtung des ersten Abschnitts (54) mit einem kleinen Durchmesser vorgesehen ist und der einen Durchmesser hat, der größer ist als derjenige des ersten Abschnitts (54) mit einem kleinen Durchmesser;
ein betätigtes Element (71), das dazu vorgesehen ist, mit dem Griffabschnitt (6) verbunden zu werden und dessen betätigter Zustand dazu eingerichtet ist, zwischen einem ersten betätigten Zustand, in welchem der Griffabschnitt (6) in den geschlossenen Zustand betätigt wird, und einem zweiten betätigten Zustand, in welchem der Griffabschnitt (6) in den offenen Zustand betätigt wird, zu wechseln,
**dadurch gekennzeichnet, dass** das betätigte Element (71) einen zweiten Abschnitt (75A, 75B) mit einem kleinen Durchmesser und einen zweiten Abschnitt (73) mit einem großen Durchmesser umfasst, der an der Seite der proximalen Richtung des zweiten Abschnitts (75A, 75B) mit einem kleinen Durchmesser vorgesehen ist und der einen Durchmesser hat, der größer ist als derjenige des zweiten Abschnitts (75A, 75B) mit einem kleinen Durchmesser; und
der Behandlungsapparat (1) ferner ein Anbindungskopplungselement (61; 90) umfasst, das zwischen einem distalen Endabschnitt des linearen Griffbetätigungsabschnitts (16, 51, 52; 16A, 16B, 51, 52) und einem proximalen Endabschnitt des betätigten Elements (71) vorgesehen ist und das zwischen dem linearen Griffbetätigungsabschnitt (16, 51, 52; 16A, 16B, 51, 52) und dem betätigten Element (71) eine Kopplung herstellt, um den betätigten Zustand des betätigten Elements (71) gemäß einer Bewegung des linearen Griffbetätigungsabschnitts (16, 51, 52; 16A, 16B, 51, 52) entlang der Achse in den Längsrichtungen zu wechseln und um es dem betätigten Element (71) zu erlauben, die Richtungen um die Achse relativ zu dem linearen Griffbetätigungsabschnitt (16, 51, 52; 16A, 16B, 51, 52) zu drehen,
wobei das Anbindungskopplungselement (61; 90) ein Kopplungselement (61; 90) umfasst, in dem ein Aufnahmehohlraum (63) gebildet ist, und
das Kopplungselement (61; 90) einen Abschnitt (62, 72; 91, 95) zur Regulierung eines Bewegungsbereichs umfasst, mit dem der erste Abschnitt (53) mit einem großen Durchmesser und/oder der zweite Abschnitt (73) mit einem großen Durchmesser drehbar verbunden ist/sind, und der dazu eingerichtet ist, einen Bewegungsbereich des ersten Abschnitts (53) mit einem großen Durchmesser und/oder des zweiten Abschnitts (73) mit einem großen Durchmesser in die Längsrichtungen zu regulieren, wobei der Abschnitt (62, 72; 91, 95) zur Regulierung eines Bewegungsbereichs in Richtung des Aufnahmehohlraums (63) in einer inneren Richtung vorsteht.

2. Behandlungsapparat (1) gemäß Anspruch 1, **dadurch gekennzeichnet, dass**
der lineare Griffbetätigungsabschnitt (16, 51, 52; 16A, 16B, 51, 52) einen Griffbetätigungsdraht (16; 16A, 16B), der durch die Griffbetätigung mit dem Griffbetätigungsabschnitt (11; 81) gezogen oder losgelassen wird, und ein distales Drahtelement (51) umfasst, das an der Seite der distalen Richtung des Griffbetätigungsdrahts (16; 16A, 16B) vorgesehen ist, um an dem Griffbetätigungsdraht (16; 16A, 16B) befestigt zu sein, und das dazu eingerichtet ist, sich in Reaktion auf ein Ziehen oder Loslassen des Griffbetätigungsdrahts (16; 16A, 16B) in den Längsrichtungen zu bewegen,
das betätigte Element (71) ein sich bewegendes Element (71) ist, das mit dem Abschnitt (62, 72; 91, 95) zur Regulierung eines Bewegungsbereichs des Kopplungselements (61, 90) verbunden ist, um sich relativ zu dem Kopplungselement (61, 90) in den Längsrichtungen zu bewegen, und
das sich bewegende Element (71) dazu eingerichtet ist, in einem betätigten Zustand in den zweiten betätigten Zustand zu wechseln, wenn es sich gemäß einer Bewegung des distalen Drahtelements (51) in der distalen Richtung aus dem ersten betätigten Zustand in der distalen Richtung bewegt, und dazu eingerichtet ist, in einem betätigten Zustand in den ersten betätigten Zustand zu wechseln, wenn es sich gemäß einer Bewegung des distalen Drahtelements (51) in der proximalen Richtung aus dem zweiten betätigten Zustand in der proximalen Richtung bewegt.

3. Behandlungsapparat (1) gemäß Anspruch 2, **dadurch gekennzeichnet, dass**
der Abschnitt (62, 72; 91, 95) zur Regulierung eines Bewegungsbereichs einen ersten Kopplungsabschnitt (62; 91), mit dem das distale Drahtelement (51) verbunden ist, um sich in die Längsrichtungen zu bewegen, und einen zweiten Kopplungsabschnitt (72; 95) umfasst, mit dem das betätigte Element (71) verbunden ist, um sich mit dem distalen Behandlungsabschnitt (4) in die Richtungen um die Achse relativ zu dem distalen Drahtelement (51) und dem flexiblen Röhrenabschnitt (5) zu drehen,
der erste Kopplungsabschnitt (62; 91) einen ersten Regulierungsabschnitt (69; 92) umfasst, der dazu eingerichtet ist, einen Bewegungsbetrag des distalen Drahtelements (51) in der proximalen Richtung zu regulieren,
das distale Drahtelement (51) einen ersten Anschlagabschnitt (55) umfasst, der dazu eingerichtet ist, eine Bewegung des distalen Drahtelements (51) in der proximalen Richtung zu verhindern, indem es gegen den ersten Regulierungsabschnitt (69; 92) anschlägt,
der zweite Kopplungsabschnitt (72; 95) einen zweiten Regulierungsabschnitt (79; 96) umfasst, der dazu eingerichtet ist, einen Bewegungsbetrag des sich bewegenden Elements (71) in der distalen Richtung zu regulieren, und
das sich bewegende Element (71) einen zweiten Anschlagabschnitt (77) umfasst, der dazu eingerichtet ist, eine Bewegung des sich bewegenden Elements (71) in der distalen Richtung zu verhindern, indem es gegen den zweiten Regulierungsabschnitt (79; 96) anschlägt.

4. Behandlungsapparat (1) gemäß Anspruch 3, **dadurch gekennzeichnet, dass**
das Kopplungselement (61; 90) ein sich drehendes Element (61) ist, das an dem ersten Kopplungsabschnitt (62) mit dem distalen Drahtelement (51) verbunden ist, um sich zusammen mit dem sich bewegenden Element (71) in die Richtungen um die Achse relativ zu dem distalen Drahtelement (51) zu drehen, und
der erste Anschlagabschnitt (55) eine gekrümmte Oberfläche (57) umfasst, die dazu eingerichtet ist, gegen den ersten Regulierungsabschnitt (69) anzuschlagen, und die parallel zu der Achse (C) in den Längsrichtungen des flexiblen Röhrenabschnitts (5) einen bogenförmigen Abschnitt hat.

5. Behandlungsapparat (1) gemäß Anspruch 3, **dadurch gekennzeichnet, dass**
das Kopplungselement (61; 90) ein sich drehendes Element (61) ist, das an dem ersten Kopplungsabschnitt (62) mit dem distalen Drahtelement (51) verbunden ist, um sich zusammen mit dem sich bewegenden Element (71) in die Richtungen um die Achse relativ zu dem distalen Drahtelement (51) zu drehen, und
der erste Regulierungsabschnitt (69) eine gekrümmte Oberfläche (89) umfasst, gegen die der erste Anschlagabschnitt (55) anzuschlagen vermag, und der parallel zu der Achse (C) in den Längsrichtungen des flexiblen Röhrenabschnitts (5) einen bogenförmigen Abschnitt hat.

6. Behandlungsapparat (1) gemäß Anspruch 3, **dadurch gekennzeichnet, dass**
das Kopplungselement (61; 90) ein Befestigungselement (90) ist, das dazu vorgesehen ist, an dem flexiblen Röhrenabschnitt (5) befestigt zu werden, und mit dem das sich bewegende Element (71) mit dem zweiten Kopplungsabschnitt (95) verbunden ist, um sich in die Richtungen um die Achse zu drehen, und
der zweite Anschlagabschnitt (77) eine gekrümmte Oberfläche (97) umfasst, die dazu eingerichtet ist, gegen den zweiten Regulierungsabschnitt (96) anzuschlagen, und der parallel zu der Achse (C) in den Längsrichtungen des flexiblen Röhrenabschnitts (5) einen bogenförmigen Abschnitt hat.

7. Behandlungsapparat (1) gemäß Anspruch 3, **dadurch gekennzeichnet, dass**
das Kopplungselement (61; 90) ein Befestigungselement (90) ist, das dazu vorgesehen ist, an dem flexiblen Röhrenabschnitt (5) befestigt zu werden, und mit dem das sich bewegende Element (71) mit dem zweiten Kopplungsabschnitt (95) verbunden ist, um sich in die Richtungen um die Achse zu drehen, und
der zweite Regulierungsabschnitt (96) eine gekrümmte Oberfläche (99) umfasst, gegen die der zweite Anschlagabschnitt (77) anzuschlagen vermag, und der parallel zu der Achse (C) in den Längsrichtungen des flexiblen Röhrenabschnitts (5) einen bogenförmigen Abschnitt hat.

8. Behandlungsapparat (1) gemäß Anspruch 3, **dadurch gekennzeichnet, dass** er ferner umfasst:
einen Rotor (26), der zwischen dem distalen Behandlungsabschnitt (4) und dem flexiblen Röhrenabschnitt (5) vorgesehen ist und der dazu eingerichtet ist, sich zusammen mit dem distalen Behandlungsabschnitt (4) in die Richtungen um die Achse relativ zu dem flexiblen Röhrenabschnitt (5) zu drehen;
einen Drehbetätigungsabschnitt (12), der an der Seite der proximalen Richtung des flexiblen Röhrenabschnitts (5) vorgesehen ist und der dazu eingerichtet ist, eine Drehbetätigung des distalen Behandlungsabschnitts (4) auszuführen;
einen Drehbetätigungsdraht (21A, 21B), der ein distales Ende hat, welches an dem Rotor (26) oder an einem Abschnitt an der Seite der distalen Richtung des Rotors (26) angebracht ist, und der sich durch eine Außenfläche des Rotors (26) und ein Inneres des flexiblen Röhrenabschnitts (55) zu dem Drehbetätigungsabschnitt (12) erstreckt, wobei der Drehbetätigungsdraht (21A, 21B) dazu eingerichtet ist, den distalen Behandlungsabschnitt (4) zu drehen, indem er durch die Drehbetätigung mit dem Drehbetätigungsabschnitt (12) gezogen oder losgelassen wird; und
einen Abschnitt (100A, 100B) zur Regulierung eines Zugbetrags, der dazu eingerichtet ist, einen Zugbetrag des Drehbetätigungsdrahts (21A, 21B) zu regulieren.

9. Behandlungsapparat (1) gemäß Anspruch 8, **dadurch gekennzeichnet, dass** der Abschnitt (100A, 100B) zur Regulierung eines Zugbetrags
ein Stoppelement (101A, 101B), das in dem flexiblen Röhrenabschnitt (5) vorgesehen ist, um an dem Drehbetätigungsdraht (21A, 21B) befestigt zu werden, und das dazu eingerichtet ist, sich in die Längsrichtungen zu bewegen, wenn der Drehbetätigungsdraht (21A, 21B) gezogen oder losgelassen wird, und
einen Lagerabschnitt (102A, 102) umfasst, der an der Seite der distalen Richtung des flexiblen Röhrenabschnitts (5) vorgesehen ist, um an dem flexiblen Röhrenabschnitt (5) befestigt zu werden, und der dazu eingerichtet ist, durch einen Anschlag des Stoppelements (101A, 101B) eine Bewegung des Stoppelements (101A, 101B) in der distalen Richtung zu verhindern.

## Revendications

1. Appareil de traitement (1) comprenant :
une section (5) de tube flexible qui s'étend dans les directions longitudinales, et qui comporte un axe (C) de directions longitudinales;
une section distale (4) de traitement qui comprend une partie (6) de préhension configurée pour effectuer un mouvement d'ouverture/fermeture entre un état ouvert et un état fermé, et qui est prévue sur un côté de direction distale de la section (5) de tube flexible de façon à être rotative dans les directions autour d'un axe par rapport à la section (5) de tube flexible ;
une section (11 , 81) d'opération de préhension qui est prévue sur un côté de direction proximale de la section (5) de tube flexible, et qui est configurée pour effectuer une opération de préhension de la partie (6) de préhension ;
une partie linéaire (16, 51, 50 ; 16A, 16B, 51, 52) d'opération de préhension qui s'étend depuis la section (11 ; 81) d'opération de préhension dans la section (5) de tube flexible, et qui est déplacée le long de l'axe (C) de directions longitudinales par l'opération de préhension de la section (11 ; 81) d'opération de préhension, la partie linéaire (16, 51, 50 ; 16A, 16B, 51, 52) d'opération de préhension comprenant une première partie de petit diamètre (54), et une première partie de grand diamètre (53) qui est prévue sur le côté de direction distale de la première partie de petit diamètre (54) et qui présente un diamètre plus grand que celui de la première partie de petit diamètre (54) ;
un élément actionné (71) qui est prévu pour être connecté à la partie (6) de préhension, et dont l'état actionné est configuré pour passer entre un premier état actionné dans lequel la partie (6) de préhension est actionnée vers l'état fermé et un deuxième état actionné dans lequel la partie (6) de préhension est actionnée vers l'état ouvert,
**caractérisé en ce que** l'élément actionné (71) comprend une deuxième partie de petit diamètre (75A, 75B) et une deuxième partie de grand diamètre (73) qui est prévue sur le côté de direction proximale de la deuxième partie de petit diamètre (75A, 75B) et qui présente un diamètre plus grand que celui de la deuxième partie de petit diamètre (75A, 75B) ; et
l'appareil de traitement (1) comprend en outre un élément (61 ; 90) de couplage de jonction qui est prévu entre une partie d'extrémité distale de la partie linéaire (16, 51, 50 ; 16A, 16B, 51, 52) d'opération de préhension et une partie d'extrémité proximale de l'élément actionné (71), et qui effectue un couplage entre la partie linéaire (16, 51, 50 ; 16A, 16B, 51, 52) d'opération de préhension et l'élément actionné (71) de façon à changer l'état actionné de l'élément actionné (71) conformément au mouvement de la partie linéaire (16, 51, 50 ; 16A, 16B, 51, 52) d'opération de préhension le long de l'axe de directions longitudinales et de façon à permettre à l'élément actionné (71) de tourner dans les directions autour de l'axe par rapport à la partie linéaire (16, 51, 50 ; 16A, 16B, 51, 52) d'opération de préhension,
dans lequel l'élément (61 ; 90) de couplage de jonction comprend un élément (61 ; 90) de couplage dans lequel un creux de réception (63) est formé, et
l'élément (61 ; 90) de couplage comprend une partie (62, 72 ; 91, 95) de régulation de plage de mouvement à laquelle au moins l'une parmi la première partie de grand diamètre (53) et la deuxième partie de grand diamètre (73) est couplée en rotation, et qui est configurée pour réguler une plage de mouvement d'au moins l'une parmi la première partie de grand diamètre (53) et la deuxième partie de grand diamètre (73) dans les directions longitudinales, la partie (62, 72 ; 91, 95) de régulation de plage de mouvement faisant saillie vers le creux de réception (63) dans une direction interne.

2. Appareil de traitement (1) selon la revendication 1, **caractérisé en ce que**
la partie linéaire (16, 51, 50 ; 16A, 16B, 51, 52) d'opération de préhension comprend un câble (16 ; 16A, 16B) d'opération de préhension qui est tiré ou relâché par l'opération de préhension avec la section (11 ; 81) d'opération de préhension, et un élément distal de câble (51) qui est prévu sur le côté de direction distale du câble (16 ; 16A, 16B) d'opération de préhension de façon à être fixé au câble (16 ; 16A, 16B) d'opération de préhension et qui est configuré pour se déplacer dans les directions longitudinales sur traction ou relâchement du câble (16 ; 16A, 16B) d'opération de préhension,
l'élément actionné (71) est un élément mobile (71) qui est couplé à la partie (62, 72 ; 91, 95) de régulation de plage de mouvement de l'élément (61, 90) de couplage de façon à se déplacer dans les directions longitudinales par rapport à l'élément (61, 90) de couplage, et
l'élément mobile (71) est configuré pour passer dans un état actionné vers le deuxième état actionné lorsqu'il se déplace à partir du premier état actionné dans la direction distale conformément à un mouvement de l'élément distal (51) de câble dans la direction distale, et configuré pour passer dans l'état actionné vers le premier état actionné lorsqu'il se déplace à partir du deuxième état actionné dans la direction proximale conformément au mouvement de l'élément distal (51) de câble dans la direction proximale.

3. Appareil de traitement (1) selon la revendication 2, **caractérisé en ce que**
la partie (62, 72 ; 91, 95) de régulation de plage de mouvement comprend une première partie (62 ; 91) de couplage à laquelle l'élément distal (51) de câble est couplé de façon à se déplacer dans les directions longitudinales, et une deuxième partie (72 ; 95) de couplage à laquelle l'élément actionné (71) est couplé de façon à tourner conjointement avec la section distale (4) de traitement dans les directions autour de l'axe par rapport à l'élément distal (51) de câble et à la section (5) de tube flexible,
la première partie (62 ; 91) de couplage comprend une première partie (69 ; 92) de régulation qui est configurée pour réguler une quantité de mouvement de l'élément distal (51) de câble dans la direction proximale,
l'élément distal (51) de câble comprend une première partie (55) de butée qui est configurée pour empêcher le mouvement de l'élément distal (51) de câble dans la direction proximale en venant en butée contre la première partie (69 ; 92) de régulation,
la deuxième partie (72 ; 95) de couplage comprend une deuxième partie (79 ; 96) de régulation qui est configurée pour réguler une quantité de mouvement de l'élément mobile (71) dans la direction distale, et
l'élément mobile (71) comprend une deuxième partie (77) de butée qui est configurée pour empêcher le mouvement de l'élément mobile (71) dans la direction distale en venant en butée contre la deuxième partie (79 ; 96) de régulation.

4. Appareil de traitement (1) selon la revendication 3, **caractérisé en ce que**
l'élément (61 ; 90) de couplage est un élément rotatif (61) qui est couplé à l'élément distal (51) de câble au niveau de la première partie (62) de couplage de façon à tourner conjointement avec l'élément mobile (71) dans les directions autour de l'axe par rapport à l'élément distal (51) de câble, et
la première partie (55) de butée comprend une surface incurvée (57) qui est configurée pour venir en butée contre la première partie (69) de régulation, et qui comporte une section arquée prise parallèle à l'axe (C) de directions longitudinales de la section (5) de tube flexible.

5. Appareil de traitement (1) selon la revendication 3, **caractérisé en ce que**
l'élément (61 ; 90) de couplage est un élément rotatif (61) qui est couplé à l'élément distal (51) de câble au niveau de la première partie (62) de couplage de façon à tourner conjointement avec l'élément mobile (71) dans les directions autour de l'axe par rapport à l'élément distal (51) de câble, et
la première partie (69) de régulation comprend une surface incurvée (89) contre laquelle la première partie (55) de butée est configurée pour venir en butée, et qui comporte une section arquée prise parallèle à l'axe (C) de directions longitudinales de la section (5) de tube flexible.

6. Appareil de traitement (1) selon la revendication 3, **caractérisé en ce que**
l'élément (61 ; 90) de couplage est un élément de fixation (90) qui est prévu pour être fixé à la section (5) de tube flexible, et sur laquelle l'élément mobile (71) est couplé avec la deuxième partie (95) de couplage de façon à tourner dans les directions autour de l'axe, et
la deuxième partie (77) de butée comprend une surface incurvée (97) qui est configurée pour venir en butée contre la deuxième partie (96) de régulation, et qui comporte une section arquée prise parallèle à l'axe (C) de directions longitudinales de la section (5) de tube flexible.

7. Appareil de traitement (1) selon la revendication 3, **caractérisé en ce que**
l'élément (61 ; 90) de couplage est un élément de fixation (90) qui est prévu pour être fixé à la section (5) de tube flexible, et sur laquelle l'élément mobile (71) est couplé avec la deuxième partie (95) de couplage de façon à tourner dans les directions autour de l'axe, et
la deuxième partie (96) de régulation comprend une surface incurvée (99) contre laquelle la deuxième partie (77) de butée est configurée pour venir en butée, et qui comporte une section arquée prise parallèle à l'axe (C) de directions longitudinales de la section (5) de tube flexible.

8. Appareil de traitement (1) selon la revendication 3, **caractérisé en ce qu'**il comprend en outre :
un rotor (26) qui est prévu entre la section distale (4) de traitement et la section (5) de tube flexible, et qui est configuré pour tourner conjointement avec la section distale (4) de traitement dans les directions autour de l'axe par rapport à la section (5) de tube flexible ;
une section (12) d'opération de rotation qui est prévue sur le côté de direction proximale de la section (5) de tube flexible, et qui est configurée pour effectuer une opération de rotation de la section distale (4) de traitement ;
un câble (21A, 21B) d'opération de rotation qui comporte une extrémité distale fixée au rotor (26) ou à une partie du côté de direction distale du rotor (26), et qui s'étend vers la section (12) d'opération de rotation à travers une surface externe du rotor (26) et un intérieur de la section (5) de tube flexible, le câble (21A, 21B) d'opération de rotation étant configuré pour faire tourner la section distale (4) de traitement en étant tiré ou relâché par l'opération de rotation avec la section (12) d'opération de rotation ; et
une partie (100A, 100B) de régulation de quantité de traction configurée pour réguler une quantité de traction du câble (21A, 21B) d'opération de rotation.

9. Appareil de traitement (1) selon la revendication 8, **caractérisé en ce que** la partie (100A, 100B) de régulation de quantité de traction comprend
un élément d'arrêt (101A, 101B) qui est prévu dans la section (5) de tube flexible pour être fixé au câble (21A, 21B) d'opération de rotation, et qui est configuré pour se déplacer dans les directions longitudinales lorsque le câble (21A, 21B) d'opération de rotation est tiré ou relâché, et
une partie (102A, 102B) de support qui est prévue sur le côté de direction distale de la section (5) de tube flexible pour être fixée à la section (5) de tube flexible, et qui est configurée pour empêcher le mouvement de l'élément d'arrêt (101A, 101B) dans la direction distale par mise en butée de l'élément d'arrêt (101A, 101B).
